# EUROPEAN PATENT APPLICATION

(11) **EP 1 454 916 A1**
(43) Date of publication of application: **08.09.2004**
(21) Application number: 03024824.9
(22) Date of filing: 30.10.2003
(51) Int. Cl.: C07K 14/755, A61K 38/37, C12N 15/12

(54) **Modified cDNA factor VIII and its derivatives**

(30) Priority: 29.11.2002 EP 02026835
(71) Applicant: ZLB Behring GmbH, 35041 Marburg (DE)
(72) Inventor: Hauser, Hans-Peter, Dr., 35041 Marburg (DE); Weimer, Thomas, Dr., 35075 Gladenbach (DE); Phillips, Martin, Dr., Wayne, PA 19087 (US)

(57) **Abstract**

Modified human factor VIII cDNA wherein mutations are inserted either in the wild-type factor VIII cDNA or in a factor VIII cDNA in which the B-domain is partially or completely deleted and may be replaced by a DNA linker segment and
A) one or several codons of the human factor VIII cDNA which are not identical with the corresponding codon in the same position of the porcine factor VIII cDNA are substituted by a different codon in such a way that
   - when the human sequence contains a codon for a neutral amino acid whereas the porcine sequence contains a codon for a charged amino acid then a codon for an amino acid with the same charge as found in the porcine sequence is introduced into the human sequence;
   - when the human sequence contains a codon for a charged amino acid whereas the porcine sequence contains a codon for a neutral amino acid then a codon for a neutral amino acid or a codon for an amino acid of t he opposite charge is introduced into the human sequence,
   - when the human sequence contains a codon for a charged amino acid whereas the porcine sequence contains a codon for an amino acid with the opposite charge then a codon for an amino acid with the opposite charge is introduced into the human sequence or
B) one or several codons for a charged amino acid which are found in the FVIII cDNA of a hemophilic patient are replaced by a codon for an amino acid of the opposite charge.

## Description

The present invention relates to modified DNA sequences coding for biologically active recombinant human factor VIII and its derivatives with improved stability, recombinant expression vectors containing such DNA sequences, host cells transformed with such recombinant expression vectors, and processes for the manufacture of the recombinant human factor VIII and its derivatives. The invention also covers a transfer vector for use in human gene therapy which comprises such modified DNA sequences.

Classic hemophilia or hemophilia A is the most common of the inherited bleeding disorders. It results from a chromosome X-linked deficiency of blood coagulation factor VIII, and affects almost exclusively males with an incidence of between one and two individuals per 10.000. The X-chromosome defect is transmitted by female carriers who are not themselves hemophiliacs. The clinical manifestation of hemophilia A is an abnormal bleeding tendency and before treatment with factor VIII concentrates was introduced the mean life span for a person with severe hemophilia was less than 20 years. The use of concentrates of factor VIII from plasma has considerably improved the situation for the hemophilia patients. The mean life span has increased extensively, giving most of them the possibility to live a more o r less normal life. However, there h ave been certain problems with the plasma derived concentrates and their use, the most serious of which have been the transmission of viruses. So far, viruses causing AIDS, hepatitis B, and non A non B hepatitis have hit the population seriously. Although different virus inactivation methods and new highly purified factor VIII concentrates have recently been developed an inadvertant contamination can not be excluded. Also, the factor VIII concentrates are fairly expensive because of the limited supply of human plasma raw material.

A factor VIII product derived from recombinant m aterial is likely to solve a large extent of the problems associated with the use of plasma derived factor VIII concentrates for treatment for hemophilia A. However, the development of a recombinant factor VIII has met some difficulties, for instance the problem of achieving production levels in sufficiently high yields, in particular regarding the full-length molecule.

In fresh plasma prepared in the presence of protease inhibitors, factor VIII has been shown to have a molecular weight of 280 kDa and to be composed of two polypeptide chains of 200 kDa and 80 kDa, respectively (Andersson, L.-O., et al. (1986) Proc. Natl. Acad. Sci. USA 83, 2979-2983). These chains are held together by metal ion bridges. More or less proteolytically degraded forms of the factor VIII molecule can be found as active fragments in factor VIII material purified from commercial concentrates (Andersson, L.-O., et al. ibid.; Andersson, L.-O., et al. (1985) EP 0 197 901). The fragmented form of factor VIII having molecular weights from 260 kDa down to 170 kDa, consists of one heavy chain with a molecular weight ranging from 180 kDa down to 90 kDa, where all variants have identical amino termini, in combination with one 80 kDa light chain. The amino-terminal region of the heavy chain is identical to that of the single chain factor VIII polypeptide that can be deduced from the nucleotide sequence data of the factor VIII cDNA (Wood, W.I., et al. (1984) Nature 312, 330-336; Vehar, G.A., et al. (1984) Nature 312, 337-342).

The smallest active form of factor VIII with a molecular weight of 170 kDa, consisting of one 90 kDa and one 80 kDa chain, can be activated with thrombin to the same extent as the higher molecular weight forms, and thus represents an unactivated form. It has also been shown to have full biological activity in vivo as tested in hemophilia dogs (Brinkhous, K.M., et al. (1985) Proc. Natl. Acad. Sci. USA 82, 8752-8756). Thus, the haemostatic effectiveness of the 170 k Da form is the same as for the high molecular weight forms of factor VIII.

The fact that the middle heavily glycosylated region of the factor VIII polypeptide chain residing between amino acids Arg-740 and Glu-1649 does not seem to be necessary for full biological activity has prompted several researchers to attempt to produce derivatives of recombinant factor VIII lacking this region. This has been achieved by deleting a portion of the cDNA encoding the middle heavily glycosylated region of factor VIII either entirely or partially.

For example, J.J. Toole, et al, reported the construction and expression of factor VIII lacking amino acids 982 through 1562, and 760 through 1639 respectively (Proc. Natl. Acad. Sci. USA (1986) 83, 5939-5942). D.L. Eaton, et al. reported the construction a nd expression of factor VIII lacking amino acids 797 through 1 562 (Blochemistry (1986) 25, 8343-8347). R.J. Kaufman described the expression of factor VIII lacking amino acids 741 through 1646 (PCT application No. WO 87/04187). N. Sarver, et al. reported the construction and expression of factor VIII lacking amino acids 747 through 1560 (DNA (1987) 6, 553-564). M. Pasek reported the construction and expression of factor VIII lacking amino acids 745 through 1562, and amino acids 741 through 1648, respectively (PCT application No. WO 88/00831). K.-D. Langner reported the construction and expression of factor VIII lacking amino acids 816 through 1598, and amino acids 741 through 1689, respectively (Behring Inst. Mitt., (1988) No. 82, 16-25, EP 0 295 597). P. Meulien, et al., reported the construction and expression of factor VIII lacking amino acids 868 through 1562, and amino acids 771 through 1666, respectively (Protein Engineering (1988) 2(4), 301-306, EP 0 303 540). When expressing these deleted forms of factor VIII cDNA in mammalian cells the production level is typically 10 times higher as compared to full-length factor VIII.

FVIII is secreted into plasma as a heterodimer of a heavy chain (domains A1-A2-B) and a light chain (A3-C1-C2) associated through a noncovalent divalent metal ion linkage between the A1- and A3 domains. In plasma, FVIII is stabilized by binding to von Willebrand factor.

Upon proteolytic activation by thrombin, FVIII is activated to a heterotrimer of 2 heavy chain fragments (A1, a 50 kDa fragment, and A2 a 43 kDa fragment) and the light chain (A3-C1-C2, a 73 kDa fragment). The active form of FVIII (FVIIIa) thus consists of a n A1-subunit associated through the d ivalent metal ion linkage to a thrombin-cleaved A3-C1-C2 light chain and a free A2 subunit associated with the A1 domain . The dissociation of that free A2 subunit from the heterotrimer is thought to be the rate limiting step in FVIIIa inactivation after thrombin activation (Fay, P.J. et al, J. Biol. Chem. 266: 8957 (1991), Fay PJ & Smudzin TM, J. Biol. Chem. 267: 13246-50 (1992)). The half life of FVIIIa in plasma is only 2.1 minutes (Saenko et al., Vox Sang. 83: 89-96 (2002)). To enhance the half life of FVIIIa would result into a longer acting FVIIIa which would also translate into less frequent injections of such a FVIII preparation. The inactivation of FVIIIa through activated Protein C (APC) by cleavage at Arg336 and Arg562 is thought not to be the rate limiting step. Attempts have been made to create a FVIIIa which is inactivation resistant by covalently attaching the A2 domain to the A3 domain and by mutating the APC cleavage sites (Pipe and Kaufman, PNAS, 94:11851-11856). However such a FVIIIa could have a thrombogenic potential as it is almost completely inactivation resistant. It is therefore the purpose of this invention to create a FVIIIa in which the A2 domain is stabilized without completely blocking inactivation.

FVIII is administered i.v. to haemophilia patients who are on prophylactic treatment about 3 times per week due to the plasma half life of FVIII of about 12 hours. It would thus be highly desirable to create a FVIII with enhanced plasma half life which could lead to a FVIII preparation which has to be administered less frequently. The present invention offers a solution to this problem by a modified FVIII molecule with an increased association of A2 to the A1/A3-C1-C2.

The nature of these modifications was identified by comparing the sequence of porcine FVIII to that of human FVIII as it is known that the dissociation of human A2 domain is threefold enhanced versus that of porcine A2 (Lollar et al., J Biol. Chem., 267:23652-23657 (1992)). The sequence comparison (Fig. 1) revealed several differences. A subset of these differences consists of differently charged amino acids. Mutants of human FVIII were constructed according to the following guidelines. When the human sequence contained a neutral amino acid whereas the porcine sequence contained a charged amino acid then a charged amino acid with the same charge as found in the porcine sequence was introduced into the human sequence. When the human sequence contained a charged amino acid whereas the porcine FVIII contained a neutral amino acid then a neutral amino acid or an amino acid of the opposite charge was introduced, e.g. if the human FVIII contained an acidic amino acid at a position where the porcine FVIII contained a neutral amino acid, also a basic amino acid was introduced. When the human sequence contained a charged sequence whereas the porcine FVIII contained a charged amino acid then an amino acid with the same charge as found in the porcine amino acid was introduced into the human sequence. Examples for such mutations which lead to an improved FVIII with a plasma half life of its activated form of more than three minutes, preferably of more than 10 minutes even more preferably more than 30 minutes, are listed in figure 2.

Other mutations for an improved FVIII were deduced by analyzing mutations in human FVIII which occurred naturally and which lead to a faster dissociation of the A2 domain associated with hemophilia. Such mutations result in differences between the two-stage assay as compared to the one-stage assay while determining FVIII clotting activity, whereas the two-stage assay result is lower than that of the one-stage assay as in the two-stage assay an incubation time of several minutes allows an unstable A2 domain to dissociate (Saenko et al., Vox Sang., 83:89-96 (2002). It was inferred that in those cases where such an increased instability was the result of the introduction of a charged amino acid that amino acid should be mutated into one of the opposite charge. Examples for such mutations which lead to an improved FVIII with a plasma half life of its activated form of more than three minutes, preferably of more than 10 minutes, even more preferably more than 30 minutes, are listed in figure 3.

As a basis for introducing the mutations preferably a modified factor VIII cDNA is used which comprises a first DNA segment coding for the amino acids 1 through 740 of the human factor VIII and a second DNA segment coding for the amino acids 1649 through 2332 of the human factor VIII. These two segments may be interconnected by a linker DNA segment, but the invention also encompasses introducing the mutations into full length FVIII.
Subject of t he i nvention is t herefore a modified h uman factor V III cDNA wherein mutations are inserted either in the wild-type factor VIII cDNA or in a factor VIII cDNA in which the B-domain is partially or completely deleted and may be replaced by a DNA linker segment, and
A) one or several codons of the human factor VIII cDNA which are not identical with the corresponding codon in the same position of the porcine factor VIII cDNA are substituted by a different codon in such a way that
   - when the human sequence contains a codon for a neutral amino acid whereas the porcine sequence contains a codon for a charged amino acid then a codon for an amino acid with the same charge as found in the porcine sequence is introduced into the human sequence;
   - when the human sequence contains a codon for a charged amino acid whereas the porcine sequence contains a codon for a neutral amino acid then a codon for a neutral amino acid or a codon for an amino acid of the opposite charge is introduced into the human sequence
   - when the human sequence contains a codon for a charged amino acid whereas the porcine sequence contains a codon for an amino acid with the opposite charge then a codon for an a mino a cid with the opposite charge is introduced into the human sequence or
B) one or several codons for a charged amino acid which are found in the FVIII cDNA of a hemophilic p atient a re replaced by a codon for an amino acid of the opposite charge.

The production of factor VIII proteins at high levels in suitable host cells, requires the assembly of the above-mentioned modified factor VIII DNA's into efficient transcriptional units together with suitable regulatory elements in a recombinant expression vector, that can be propagated in E. coli according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the factor VIII DNA's, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The factor VIII cDNA's are then integrated into the genome into a suitable host cell line for expression of the factor VIII proteins. Preferably this cell line should be an animal cell-line of vertebrate origin in order to ensure correct folding, disulfide bond formation, asparagines-linked glycosylation and other post-translational modifications as well as secretion into the cultivation medium. Examples on other post-translational modifications are tyrosine O-sulfation, and proteolytic processing of the nascent polypeptide chain. Examples of cell lines that can be use are monkey COS-cells, mouse L-cells, mouse C127-cells, hamster BHK-21 cells, human embryonic kidney 293 cells, and preferentially CHO-cells.

The recombinant expression vector encoding the factor VIII cDNA's can be introduced into an animal cell line in several different ways. For instance, recombinant expression vectors can be created from vectors based on different animal viruses, Examples of these are vectors based on baculovirus, vaccinia virus, adenovirus, and preferably bovine papilloma virus.

The transcription units encoding the factor VIII DNA's can also be introduced into animal cells together with another recombinant gene which may function as a dominant selectable marker in these cells in order to facilitate the isolation of specific cell clones which have integrated the recombinant DNA into their genome. Examples of this type of dominant selectable marker genes are Tn5 aminoglycoside phosphotransferase, conferring resistance to Geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the factor VIII cDNA, or it can be encoded on a separate vector which is simultaneously introduced and integrated to the genome of the host cell, frequently resulting in a tight physical linkage between the different transcription units.

Other types of selectable marker genes which can be used together with the factor VIII DNA's are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthin, thymidin, and glycine. These dhfr-genes can be introduced together with the factor VIII cDNA transcriptional units into CHO-cells of the above type, either linked on the same vector or on different vectors, thus creating dhfr-positive cell lines producing recombinant factor VIII protein.

If the above cell lines are grown in the presence of the cytotoxic dhfr-inhibitor methotrexate, new cell lines resistant to methotrexate will emerge. These cell lines may produce recombinant factor VIII protein at an increased rate due to the amplified number of linked dhfr and factor VIII transcriptional units. When propagating these cell lines in increasing concentrations of methotrexate (1-10000 nM), new cell lines can be obtained which produce factor VIII protein at very high rate.

The above cell lines producing factor VIII protein can be grown on a large scale, either in suspension culture or on various solid supports. Examples of these supports are microcarriers based on dextran or collagen matrices, or solid supports in the form of hollow fibres or various ceramic materials. When grown in cell suspension culture or on microcarriers the culture of the above cell lines can be performed either as a bath culture or as a perfusion culture with continuous production of conditioned medium over extended periods of time. Thus, according to the present invention, the above cell lines are well suited for the development of an industrial process for the production of recombinant factor VIII that can be isolated from human plasma.

The recombinant factor VIII protein which accumulate in the medium of CHO-cells of the above type, can be concentrated and purified by a variety of biochemical and chromatographic methods, including methods utilizing differences in size, charge, hydrophobicity, solubility, specific affinity, etc. between the recombinant factor VIII protein and other substances in the cell cultivation medium.

An example of such a purification is the adsorption of the recombinant factor VIII protein to a monoclonal antibody which is immobilised on a solid support. After desorption, the factor VIII protein can be further purified by a variety of chromatographic techniques based on the above properties.

The recombinant proteins with factor VIII activity described in this invention can be formulated into pharmaceutical preparations for therapeutic use. The purified factor VIII proteins may be dissolved in conventional physiologically compatible aqueous buffer solutions to which there may be added, optionally, pharmaceutical adjuvants to provide pharmaceutical preparations.

The modified factor VIII DNA's of this invention may also be integrated into a transfer vector for use in the human gene therapy.

A further subject of this invention is a modified biologically active recombinant human factor VIII with improved plasma half life of its activated form wherein mutations are inserted either in the wild-type factor VIII or in a FVIII in which the B-domain is partially or completely deleted and replaced by a linker, and
A) one or several amino acids of the human factor VIII which are not identical with the corresponding amino acid in the same position of the porcine factor VIII are substituted by a different amino acid in such a way that
   - when the human sequence contains a neutral amino acid whereas the porcine sequence contains a charged amino acid then a charged amino acid with the same charge as found in the porcine sequence is introduced into the human sequence;
   - when the human sequence contains a charged amino acid whereas the porcine sequence contains a neutral amino acid then a neutral amino acid or an amino acid of the opposite charge is introduced into the human sequence;
   - when the human sequence contains a charged amino acid whereas the porcine sequence contains an amino acid with the opposite charge then an amino acid with the opposite charge is introduced into the human sequence or
B) one or several charged amino acids which are found in the FVIII amino sequence of hemophilic patients are replaced by a codon for an amino acid of the opposite charge.

The present invention will be further described more in detail in the following examples thereof. This description of specific embodiments of the invention will be made in conjunction with the appended figures.

### Generation of FVIII mutants

For the generation of FVIII mutants, a suitable subfragment of the FVIII cDNA (e.g. Aval - Sacl, encompassing aminoacids 226 to 978) is first subcloned into a suitable cloning vector to reduce subsequent sequencing efforts. Site directed mutagenesis is then performed with a commercially available mutagenesis kit (e.g. QuickChange SiteDirected Mutagenesis Kit (Stratagene) according to the manufacturer's instructions. Primers used for mutagenesis are listed in the attached sequence listing and below, where the mutagenic bases are indicated in bold letters.
Mutation: A284K
Forward primer 5'GGAACCATCGCCAGAAGTCCTTGGAAATCTCGCC3'     (Sequence 1) Reverse primer 5'GGCGAGATTTCCAAGGACTTCTGGCGATGGTTCC3' (Sequence 2) Mutation: D318G
Forward primer 5'CCCACCAACATGGTGGCATGGAAGCTTATGTC3'    (Sequence 3) Reverse primer 5'GACATAAGCTTCCATGCCACCATGTTGGTGGG3' (Sequence 4) Mutation: M337R 
Forward primer
5'CAGAGGAACCCCAACTACGACGTAAAAATAATGAAGAAGCGGAAGAC3'
(Sequence 5)
 
Reverse primer
5'GTCTTCCGCTTCTTCATTATTTTTACGTCGTAGTTGGGGTTCCTCTG3'
(Sequence 6)
 Mutation: N340D 
Forward primer
5'CCCAACTACGAATGAAAAATGATGAAGAAGCGGAAGACTATG3'
(Sequence 7)
 
Reverse primer
5'CATAGTCTTCCGCTTCTTCATCATTTTTCATTCGTAGTTGGG3'
(Sequence 8)
 Mutation: D349N 
Forward primer
5'GAAGAAGCGGAAGACTATGATGATAATCTTACTGATTCTG3'
(Sequence 9)
 
Reverse primer 5'CAGAATCAGTAAGATTATCATCATAGTCTTCCGCTTCTTC3'
(Sequence 10)
 Mutation: N364D 
Forward primer 5′GGTCAGGTTTGATGATGACGACTCTCCTTCCTTTATCC3′
(Sequence 11)
 
Reverse primer 5'GGATAAAGGAAGGAGAGTCGTCATCATCAAACCTGACC3'
(Sequence 12)
 Mutation: D403S 
Forward primer 5'CCCTTAGTCCTCGCCCCCTCTGACAGAAGTTATAAAAG3'
(Sequence 13)
 
Reverse primer 5´CTTTTATAACTTCTGTCAGAGGGGGCGAGGACTAAGGG3´
(Sequence 14)
 Mutation: E434V 
Forward primer
5'GTCCGATTTATGGCATACACAGATGTTACCTTTAAGACTCG3'
(Sequence 15)
 
Reverse primer
5´CGAGTCTTAAAGGTAACATCTGTGTATGCCATAAATCGGAC3´
(Sequence 16)
 Mutation: E440K 
Forward primer
5´CCTTTAAGACTCGTAAAGCTATTCAGCATGAATCAGG3´ (Sequence 17)
 
Reverse primer
5´CCTGATTCATGCTGAATAGCTTTACGAGTCTTAAAGG3´ (Sequence 18)
 Mutation: Q468K 
Forward primer
5'CACACTGTTGATTATATTTAAGAATAAAGCAAGCAGACCATATAAC3'
(Sequence 19)
 
Reverse primer
5´GTTATATGGTCTGCTTGCTTTATTCTTAAATATAATCAACAGTGTG3´
(Sequence 20)
 Mutation: R484S 
Forward primer
5'CCCTCACGGAATCACTGATGTCTCTCCTTTGTATTCAAGG3'
(Sequence 21)
 
Reverse primer
5´CCTTGAATACAAAGGAGAGACATCAGTGATTCCGTGAGGG3´
(Sequence 22)
 Mutation: R489G 
Forward primer 5'GATGTCCGTCCTTTGTATTCAGGGAGATTACCAAAAGG3'
(Sequence 23)
 
Reverse primer 5'CCTTTTGGTAATCTCCCTGAATACAAAGGACGGACATC3'
(Sequence 24)
 Mutation: R583Q 
Forward primer
5´CTGTATTTGATGAGAACCAAAGCTGGTACCTCACAG3´ (Sequence 25)
 
Reverse primer
5'CTGTGAGGTACCAGCTTTGGTTCTCATCAAATACAG3' (Sequence 26)
 Mutation: A599D 
Forward primer
5'CTCCCCAATCCAGATGGAGTGCAGCTTGAG3'          (Sequence 27)
 
Reverse primer
5'CTCAAGCTGCACTCCATCTGGATTGGGGAG3'          (Sequence 28)
 Mutation: E604Q 
Forward primer
5'CAGCTGGAGTGCAGCTTCAGGATCCAGAGTTC3' (Sequence 29)
 
Reverse primer
5'GAACTCTGGATCCTGAAGCTGCACTCCAGCTG3'          (Sequence 30)
 Mutation: G1948K

Forward primer
5′CGATGGTATCTGCTCAGCATGAAGAGCAATGAAAACATCCATTCTATT
C3′ (Sequence 31)

Reverse primer
5′GAATAGAATGGATGTTTTCATTGCTCTTCATGCTGAGCAGATACCATCG
3′ (Sequence 32)

After clone isolation and sequence verification mutant subfragments are reinserted into the respective expression vector.

### Expression of FVIII mutants

Transfection of FVIII mutant clones and expression of the mutant FVIII molecules is done as described previously and known to those skilled in the art (e.g. Plantier JL et al. Thromb. Haemost. 86:596-603 (2001 )).

### Measuring affinity of A2 subunit for A1/A3-C1-C2

The increased affinity of the A2 subunit for the A1/A3-C1-C2 can be measured as previously described by functional assays (Fay PJ & Smudzin TM. J. Biol. Chem. 267:13246-50 (1992); Lollar P et al. J. Biol. Chem. 267:23652-57 (1992)) as well as a physical assay employing surface plasmon resonance (Persson E et al. Biochemistry 34:12775-81 (1995)).

The sequence of the porcine factor VIII is shown in Sequence 33, whereas the sequence of the human factor VIII is shown in sequence 34 of the attached sequence listing.

Fig. 1: Comparison of porcine to human FVIII protein sequence (numbers refer to mature human FVIII; in the porcine sequence, only discrepant aminoacids are displayed; identical aminoacids are indicated by a ".")

241 . . . . . . . . . . K . . . . . . . . . Porcine FVIII
240 R S L P G L I G C H R K S V Y W H V I G Human FVIII

261 . . . S . . . . . . . . . . . . . . . . Porcine FVIII
260 M G T T P E V H S I F L E G H T F L V R Human FVIII

281 H . . . . . . . . . . L . . . . . . . F Porcine FVIII
280 N H R Q A S L E I S P I T F L T A Q T L Human FVIII

301 . . . . . . . . . . . . . . . . H . G . Porcine FVIII
300 L M D L G Q F L L F C H I S S H Q H D G Human FVIII

321 . . . H . R . E . . A . . . . . . R . A Porcine FVIII
320 M E A Y V K V D S C P E E P Q L R M K N Human FVIII

341 D . . - . . . . . N . Y . . D . . . . . Porcine FVIII
340 N E E A E D Y D D D L T D S E M D V V R Human FVIII

360 L . G . D V S P . . . . . . . . . . . . Porcine FVIII
360 F D D D N S P S F I Q I R S V A K K H P Human FVIII

380 . . . . . . . S . . . . . . . . . . A . Porcine FVIII
380 K T W V H Y I A A E E E D W D Y A P L V Human FVIII

400 P S . S . . . . . . L . . . S . . . . . Porcine FVIII
400 L A P D D R S Y K S Q Y L N N G P Q R I Human FVIII

420 . . . . . . A . . V . . . . V . . . . . Porcine FVIII
420 G R K Y K K V R F M A Y T D E T F K T R Human FVIII

440 K . . P Y . . . . . . . . . . . . . . . Porcine FVIII
440 E A I Q H E S G I L G P L L Y G E V G D Human FVIII

460 . . . . . . . . K . . . . . . . . . . . Porcine FVIII
460 T L L I I F K N Q A S R P Y N I Y P H G Human FVIII

480 . . . . S A . H P G . . L . . W . . . . Porcine FVIII
480 I T D V R P L Y S R R L P K G V K H L K Human FVIII

500 . M . . . . . . T . . . . . . . . . . . Porcine FVIII
500 D F P I L P G E I F K Y K W T V T V E D Human FVIII

520 . . . . . . . . . . . . . . . . S I . L Porcine FVIII
520 G P T K S D P R C L T R Y Y S S F V N M Human FVIII

540 . K . . . . . . . . . . . . . . . . . . Porcine FVIII
540 E R D L A S G L I G P L L I C Y K E S V Human FVIII

560 . . . . . . M . . . . . . . . . . . . . Porcine FVIII
560 D Q R G N Q I M S D K R N V I L F S V F Human FVIII

580 . . . Q . . . . A . . . . . . . . . . D Porcine FVIII
580 D E N R S W Y L T E N I Q R F L P N P A Human FVIII

600 . L . P Q . . . . . . . . . . . . . . . Porcine FVIII
600 G V Q L E D P E F Q A S N I M H S I N G Human FVIII

**Fig. 2: Mutations of human FVIII which are based on a protein comparison with porcine FVIII (does not comprise all mutations which are possible according to the invention):**

| aa (mature human FVIII) | Amino acid in human sequence | Mutated to | Or mutated to |
|---|---|---|---|
| 318 | D | G | K |
| 337 | M | R | |
| 340 | N | D | |
| 349 | D | N | K |
| 364 | N | D | |
| 403 | D | S | K |
| 434 | E | V | K |
| 440 | E | K | |
| 468 | Q | K | |
| 484 | R | S | E |
| 489 | R | G | E |
| 583 | R | Q | E |
| 599 | A | D | |
| 604 | E | Q | K |

**Fig. 3: Mutations of human FVIII which are based on an analysis of existing human mutations leading to an enhanced dissociation of the A2 domain (does not comprise all mutations possible according to the invention)**

| aa (Mature human FVIII) | From (human sequence) | To | Comments |
|---|---|---|---|
| 284 | A | K | A to E leads to enhanced dissociation of A2 domain |
| 1948 | G | K | G to D leads to enhanced dissociation of A2 domain |

In the following Sequence Listing Sequences 1-32 describe oligonucleotides which are used to introduce specific mutations into FVIII. Sequence 33 is the amino acid sequence of full length mature porcine FVIII, Sequence 34 is the amino acid sequence of full length mature human FVIII.

Sequence Listing
Organization Applicant
----------------------
Street : Emil-von-Behring-Straße 76
City : Marburg
State : Hessen
Country : Germany
PostalCode : 35041
PhoneNumber : 0049-6421-39 45 06
FaxNumber : 0049-6421-39 45 58
EmailAddress : Hugo.Pfeil@aventis.com
<110> OrganizationName : Aventis Behring GmbH

Application Project
-------------------
<120> Title : Modified cDNA Factor VIII and its Derivatives
<130> AppFileReference : 2002/M018 (A66)
<140> CurrentAppNumber :
<141> CurrentFilingDate : ____-__-__

Sequence
--------
<213> OrganismName : Artificial Sequence
<400> PreSequenceString :
ggaaccatcg ccagaagtcc ttggaaatct cgcc 34
<212> Type : DNA
<211> Length : 34
SequenceName : Sequenz 1
SequenceDescription :

Feature
-------
Sequence: Sequenz 1:
<221> FeatureKey : primer_bind
<222> LocationFrom : 1
<222> LocationTo : 34
Other Information :
CDSJoin : No

Custom Codon
Sequence Name : Sequenz 1

Sequence
--------
<213> OrganismName : Artificial Sequence
<400> PreSequenceString :
ggcgagattt ccaaggactt ctggcgatgg ttcc 34
<212> Type : DNA
<211> Length : 34
SequenceName : Sequenz 2
SequenceDescription :

Feature
-------
Sequence: Sequenz 2:
<221> FeatureKey : primer_bind
<222> LocationFrom : 1
<222> LocationTo : 34
Other Information :
CDSJoin : No

Custom Codon
------------
Sequence Name : Sequenz 2

Sequence
--------
<213> OrganismName : Artificial Sequence
<400> PreSequenceString :
cccaccaaca tggtggcatg gaagcttatg tc 32
<212> Type : DNA
<211> Length : 32
SequenceName : Sequenz 3
SequenceDescription :

Feature
-------

Sequence: Sequenz 3:
<221> FeatureKey : primer_bind
<222> LocationFrom : 1
<222> LocationTo : 32
Other Information :
CDSJoin : No

Custom Codon
------------
Sequence Name : Sequenz 3

Sequence
--------
<213> OrganismName : Artificial Sequence
<400> PreSequenceString :
gacataagct tccatgccac catgttggtg gg 32
<212> Type : DNA
<211> Length : 32
SequenceName : Sequenz 4
SequenceDescription :

Feature
-------
Sequence: Sequenz 4:
<221> FeatureKey : primer_bind
<222> LocationFrom : 1
<222> LocationTo : 32
Other Information :
CDSJoin : No

Custom Codon
------------
Sequence Name : Sequenz 4

Sequence
--------
<213> OrganismName : Artificial Sequence
<400> PreSequenceString :

cagaggaacc ccaactacga cgtaaaaata atgaagaagc ggaagac 47
<212> Type : DNA
<211> Length : 47
SequenceName : Sequenz 5
SequenceDescription :

Feature
-------
Sequence: Sequenz 5:
<221> FeatureKey : primer_bind
<222> LocationFrom : 1
<222> LocationTo : 47
Other Information :
CDSJoin : No

Custom Codon
------------
Sequence Name : Sequenz 5

Sequence
--------
<213> OrganismName : Artificial Sequence
<400> PreSequenceString :
gtcttccgct tcttcattat ttttacgtcg tagttggggt tcctctg 47
<212> Type : DNA
<211> Length : 47
SequenceName : Sequenz 6
SequenceDescription :

Feature
-------
Sequence: Sequenz 6:
<221> FeatureKey : primer_bind
<222> LocationFrom : 1
<222> LocationTo : 47
Other Information :
CDSJoin : No

Custom Codon
------------
Sequence Name : Sequenz 6

Sequence
--------
<213> OrganismName : Artificial Sequence
<400> PreSequenceString :
cccaactacg aatgaaaaat gatgaagaag cggaagacta tg 42
<212> Type : DNA
<211> Length : 42
SequenceName : Sequenz 7
SequenceDescription :

Feature
-------
Sequence: Sequenz 7:
<221> FeatureKey : primer_bind
<222> LocationFrom : 1
<222> LocationTo : 42
Other Information :
CDSJoin : No

Custom Codon
------------
Sequence Name : Sequenz 7

Sequence
--------
<213> OrganismName : Artificial Sequence
<400> PreSequenceString :
catagtcttc cgcttcttca tcatttttca ttcgtagttg gg 42
<212> Type : DNA
<211> Length : 42
SequenceName : Sequenz 8
SequenceDescription :

Feature

-------
Sequence: Sequenz 8:
<221> FeatureKey : primer_bind
<222> LocationFrom : 1
<222> LocationTo : 42
Other Information :
CDSJoin : No

Custom Codon
------------
Sequence Name : Sequenz 8

Sequence
--------
<213> OrganismName : Artificial Sequence
<400> PreSequenceString :
gaagaagcgg aagactatga tgataatctt actgattctg 40
<212> Type : DNA
<211> Length : 40
SequenceName : Sequenz 9
SequenceDescription :

Feature
-------
Sequence: Sequenz 9:
<221> FeatureKey : primer_bind
<222> LocationFrom : 1
<222> LocationTo : 40
Other Information :
CDSJoin : No

Custom Codon
------------
Sequence Name : Sequenz 9

Sequence
--------
<213> OrganismName : Artificial Sequence

<400> PreSequenceString :
cagaatcagt aagattatca tcatagtctt ccgcttcttc 40
<212> Type : DNA
<211> Length : 40
SequenceName : Sequenz 10
SequenceDescription :

Feature
-------
Sequence: Sequenz 10:
<221> FeatureKey : primer_bind
<222> LocationFrom : 1
<222> LocationTo : 40
Other Information :
CDSJoin : No

Custom Codon
------------
Sequence Name : Sequenz 10

Sequence
--------
<213> OrganismName : Artificial Sequence
<400> PreSequenceString :
ggtcaggttt gatgatgacg actctccttc ctttatcc 38
<212> Type : DNA
<211> Length : 38
SequenceName : Sequenz 11
SequenceDescription :

Feature
-------
Sequence: Sequenz 11:
<221> FeatureKey : primer_bind
<222> LocationFrom : 1
<222> LocationTo : 38
Other Information :
CDSJoin : No

Custom Codon
------------
Sequence Name : Sequenz 11

Sequence
--------
<213> OrganismName : Artificial Sequence
<400> PreSequenceString :
ggataaagga aggagagtcg tcatcatcaa acctgacc 38
<212> Type : DNA
<211> Length : 38
SequenceName : Sequenz 12
SequenceDescription :

Feature
-------
Sequence: Sequenz 12:
<221> FeatureKey : primer_bind
<222> LocationFrom : 1
<222> LocationTo : 38
Other Information :
CDSJoin : No

Custom Codon
------------
Sequence Name : Sequenz 12

Sequence
--------
<213> OrganismName : Artificial Sequence
<400> PreSequenceString :
cccttagtcc tcgcccctc tgacagaagt tataaaag 38
<212> Type : DNA
<211> Length : 38
SequenceName : Sequenz 13
SequenceDescription :

Feature
-------
Sequence: Sequenz 13:
<221> FeatureKey : primer_bind
<222> LocationFrom : 1
<222> LocationTo : 38
Other Information :
CDSJoin : No

Custom Codon
------------
Sequence Name : Sequenz 13

Sequence
--------
<213> OrganismName : Artificial Sequence
<400> PreSequenceString :
cttttataac ttctgtcaga gggggcgagg actaaggg 38
<212> Type : DNA
<211> Length : 38
SequenceName : Sequenz 14
SequenceDescription :

Feature
-------
Sequence: Sequenz 14:
<221> FeatureKey : primer_bind
<222> LocationFrom : 1
<222> LocationTo : 38
Other Information :
CDSJoin : No

Custom Codon
------------
Sequence Name : Sequenz 14

Sequence
--------

<213> OrganismName : Artificial Sequence
<400> PreSequenceString :
gtccgattta tggcatacac agatgttacc tttaagactc g 41
<212> Type : DNA
<211> Length : 41
SequenceName : Sequenz 15
SequenceDescription :

Feature
-------
Sequence: Sequenz 15:
<221> FeatureKey : primer_bind
<222> LocationFrom : 1
<222> LocationTo : 41
Other Information :
CDSJoin : No

Custom Codon
------------
Sequence Name : Sequenz 15

Sequence
--------
<213> OrganismName : Artificial Sequence
<400> PreSequenceString :
cgagtcttaa aggtaacatc tgtgtatgcc ataaatcgga c 41
<212> Type : DNA
<211> Length : 41
SequenceName : Sequenz 16
SequenceDescription :

Feature
-------
Sequence: Sequenz 16:
<221> FeatureKey : primer_bind
<222> LocationFrom : 1
<222> LocationTo : 41
Other Information :

CDSJoin : No

Custom Codon
------------
Sequence Name : Sequenz 16

Sequence
--------
<213> OrganismName : Artificial Sequence
<400> PreSequenceString :
cctttaagac tcgtaaagct attcagcatg aatcagg 37
<212> Type : DNA
<211> Length : 37
SequenceName : Sequenz 17
SequenceDescription :

Feature
-------
Sequence: Sequenz 17:
<221> FeatureKey : primer_bind
<222> LocationFrom : 1
<222> LocationTo : 37
Other Information :
CDSJoin : No

Custom Codon
------------
Sequence Name : Sequenz 17

Sequence
--------
<213> OrganismName : Artificial Sequence
<400> PreSequenceString :
cctgattcat gctgaatagc tttacgagtc ttaaagg 37
<212> Type : DNA
<211> Length : 37
SequenceName : Sequenz 18
SequenceDescription :

Feature
-------
Sequence: Sequenz 18:
<221> FeatureKey : primer_bind
<222> LocationFrom : 1
<222> LocationTo : 37
Other Information :
CDSJoin : No

Custom Codon
------------
Sequence Name : Sequenz 18

Sequence
--------
<213> OrganismName : Artificial Sequence
<400> PreSequenceString :
cacactgttg attatattta agaataaagc aagcagacca tataac 46
<212> Type : DNA
<211> Length : 46
SequenceName : Sequenz 19
SequenceDescription :

Feature
-------
Sequence: Sequenz 19:
<221> FeatureKey : primer_bind
<222> LocationFrom : 1
<222> LocationTo : 46
Other Information :
CDSJoin : No

Custom Codon
------------
Sequence Name : Sequenz 19

Sequence

--------
<213> OrganismName : Artificial Sequence
<400> PreSequenceString :
gttatatggt ctgcttgctt tattcttaaa tataatcaac agtgtg 46
<212> Type : DNA
<211> Length : 46
SequenceName : Sequenz 20
SequenceDescription :

Feature
-------
Sequence: Sequenz 20:
<221> FeatureKey : primer_bind
<222> LocationFrom : 1
<222> LocationTo : 46
Other Information :
CDSJoin : No

Custom Codon
------------
Sequence Name : Sequenz 20

Sequence
--------
<213> OrganismName : Artificial Sequence
<400> PreSequenceString :
ccctcacgga atcactgatg tctctccttt gtattcaagg 40
<212> Type : DNA
<211> Length : 40
SequenceName : Sequenz 21
SequenceDescription :

Feature
-------
Sequence: Sequenz 21:
<221> FeatureKey : primer_bind
<222> LocationFrom : 1
<222> LocationTo : 40

Other Information :
CDSJoin : No

Custom Codon
------------
Sequence Name : Sequenz 21

Sequence
--------
<213> OrganismName : Artificial Sequence
<400> PreSequenceString :
ccttgaatac aaaggagaga catcagtgat tccgtgaggg 40
<212> Type : DNA
<211> Length : 40
SequenceName : Sequenz 22
SequenceDescription :

Feature
-------
Sequence: Sequenz 22:
<221> FeatureKey : primer_bind
<222> LocationFrom : 1
<222> LocationTo : 40
Other Information :
CDSJoin : No

Custom Codon
------------
Sequence Name : Sequenz 22

Sequence
--------
<213> OrganismName : Artificial Sequence
<400> PreSequenceString :
gatgtccgtc ctttgtattc agggagatta ccaaaagg 38
<212> Type : DNA
<211> Length : 38
SequenceName : Sequenz 23

SequenceDescription :

Feature
-------
Sequence: Sequenz 23:
<221> FeatureKey : primer_bind
<222> LocationFrom : 1
<222> LocationTo : 38
Other Information :
CDSJoin : No

Custom Codon
------------
Sequence Name : Sequenz 23

Sequence
--------
<213> OrganismName : Artificial Sequence
<400> PreSequenceString :
ccttttggta atctccctga atacaaagga cggacatc 38
<212> Type : DNA
<211> Length : 38
SequenceName : Sequenz 24
SequenceDescription :

Feature
-------
Sequence: Sequenz 24:
<221> FeatureKey : primer_bind
<222> LocationFrom : 1
<222> LocationTo : 38
Other Information :
CDSJoin : No

Custom Codon
------------
Sequence Name : Sequenz 24

Sequence
--------
<213> OrganismName : Artificial Sequence
<400> PreSequenceString :
ctgtatttga tgagaaccaa agctggtacc tcacag 36
<212> Type : DNA
<211> Length : 36
SequenceName : Sequenz 25
SequenceDescription :

Feature
-------
Sequence: Sequenz 25:
<221> FeatureKey : primer_bind
<222> LocationFrom : 1
<222> LocationTo : 36
Other Information :
CDSJoin : No

Custom Codon
------------
Sequence Name : Sequenz 25

Sequence
--------
<213> OrganismName : Artificial Sequence
<400> PreSequenceString :
ctgtgaggta ccagctttgg ttctcatcaa atacag 36
<212> Type : DNA
<211> Length : 36
SequenceName : Sequenz 26
SequenceDescription :

Feature
-------
Sequence: Sequenz 26:
<221> FeatureKey : primer_bind
<222> LocationFrom : 1

<222> LocationTo : 36
      Other Information :
      CDSJoin : No

Custom Codon
------------
Sequence Name : Sequenz 26

Sequence
--------
<213> OrganismName : Artificial Sequence
<400> PreSequenceString :
ctccccaatc cagatggagt gcagcttgag                                          30
<212> Type : DNA
<211> Length : 30
      SequenceName : Sequenz 27
      SequenceDescription :

Feature
-------
Sequence: Sequenz 27:
<221> FeatureKey : primer_bind
<222> LocationFrom : 1
<222> LocationTo : 30
      Other Information :
      CDSJoin : No

Custom Codon
------------
Sequence Name : Sequenz 27

Sequence
--------
<213> OrganismName : Artificial Sequence
<400> PreSequenceString :
ctcaagctgc actccatctg gattggggag                                          30
<212> Type : DNA
<211> Length : 30

SequenceName : Sequenz 28
SequenceDescription :

Feature
-------
Sequence: Sequenz 28:
<221> FeatureKey : primer_bind
<222> LocationFrom : 1
<222> LocationTo : 30
Other Information :
CDSJoin : No

Custom Codon
------------
Sequence Name : Sequenz 28

Sequence
--------
<213> OrganismName : Artificial Sequence
<400> PreSequenceString :
cagctggagt gcagcttcag gatccagagt tc 32
<212> Type : DNA
<211> Length : 32
SequenceName : Sequenz 29
SequenceDescription :

Feature
-------
Sequence: Sequenz 29:
<221> FeatureKey : primer_bind
<222> LocationFrom : 1
<222> LocationTo : 32
Other Information :
CDSJoin : No

Custom Codon
------------
Sequence Name : Sequenz 29

Sequence
--------
<213> OrganismName : Artificial Sequence
<400> PreSequenceString :
gaactctgga tcctgaagct gcactccagc tg 32
<212> Type : DNA
<211> Length : 32
SequenceName : Sequenz 30
SequenceDescription :

Feature
-------
Sequence: Sequenz 30:
<221> FeatureKey : primer_bind
<222> LocationFrom : 1
<222> LocationTo : 32
Other Information :
CDSJoin : No

Custom Codon
------------
Sequence Name : Sequenz 30

Sequence
--------
<213> OrganismName : Artificial Sequence
<400> PreSequenceString :
cgatggtatc tgctcagcat gaagagcaat gaaaacatcc attctattc 49
<212> Type : DNA
<211> Length : 49
SequenceName : Sequenz 31
SequenceDescription :

Feature
-------
Sequence: Sequenz 31:
<221> FeatureKey : primer_bind

<222> LocationFrom : 1
<222> LocationTo : 49
      Other Information :
      CDSJoin : No

Custom Codon
------------
Sequence Name : Sequenz 31

Sequence
--------
<213> OrganismName : Artificial Sequence
<400> PreSequenceString :
gaatagaatg gatgttttca ttgctcttca tgctgagcag ataccatcg          49
<212> Type : DNA
<211> Length : 49
      SequenceName : Sequenz 32
      SequenceDescription :

Feature
-------
Sequence: Sequenz 32:
<221> FeatureKey : primer_bind
<222> LocationFrom : 1
<222> LocationTo : 49
      Other Information :
      CDSJoin : No

Custom Codon
------------
Sequence Name : Sequenz 32

Sequence
--------
<213> OrganismName : Porcine
<400> PreSequenceString :
AIRRYYLGAV ELSWDYRQSE LLRELHVDTR FPATAPGALP LGPSVLYKKT VFVEFTDQLF          60

SVARPRPPWM GLLGPTIQAE VYDTVVVTLK NMASHPVSLH AVGVSFWKSS EGAEYEDHTS 120

QREKEDDKVL PGKSQTYVWQ VLKENGPTAS DPPCLTYSYL SHVDLVKDLN SGLIGALLVC 180

REGSLTRERT QNLHEFVLLF AVFDEGKSWH SARNDSWTRA MDPAPARAQP AMHTVNGYVN 240

RSLPGLIGCH KKSVYWHVIG MGTSPEVHSI FLEGHTFLVR HHRQASLEIS PLTFLTAQTF 300

LMDLGQFLLF CHISSHHHGG MEAHVRVESC AEEPQLRRKA DEEEDYDDNL YDSDMDVVRL 360

DGDDVSPFIQ IRSVAKKHPK TWVHYISAEE EDWDYAPAVP SPSDRSYKSL YLNSGPQRIG 420

RKYKKARFVA YTDVTFKTRK AIPYESGILG PLLYGEVGDT LLIIFKNKAS RPYNIYPHGI 480

TDVSALHPGR LLKGWKHLKD MPILPGETFK YKWTVTVEDG PTKSDPRCLT RYYSSSINLE 540

KDLASGLIGP LLICYKESVD QRGNQMMSDK RNVILFSVFD ENQSWYLAEN IQRFLPNPDG 600

LQPQDPEFQA SNIMHSINGY VFDSLQLSVC LHEVAYWYIL SVGAQTDFLS VFFSGYTFKH 660

KMVYEDTLTL FPFSGETVFM SMENPGLWVL GCHNSDLRNR GMTALLKVYS CDRDIGDYYD 720

NTYEDIPGFL LSGKNVIEPR SFAQNSRPPS ASQKQFQTIT SPEDDVELDP QSGERTQALE 780

ELSVPSGDGS MLLGQNPAPH GSSSSDLQEA RNEADDYLPG ARERNTAPSA AARLRPELHH 840

SAERVLTPEP EKELKKLDSK MSSSSDLLKT SPTIPSDTLS AETERTHSLG PPHPQVNFRS 900

QLGAIVLGKN SSHFIGAGVP LGSTEEDHES SLGENVSPVE SDGIFEKERA HGPASLTKDD 960

VLFKVNISLV KTNKARVYLK TNRKIHIDDA ALLTENRASA TFMDKNTTAS GLNHVSNWIK 1020

GPLGKNPLSS ERGPSPELLT SSGSGKSVKG QSSGQGRIRV AVEEEELSKG KEMMLPNSEL 1080

TFLTNSADVQ GNDTHSQGKK SREEMERREK LVQEKVDLPQ VYTATGTKNF LRNIFHQSTE 1140

PSVEGFDGGS HAPVPQDSRS LNDSAERAET HIAHFSAIRE EAPLEAPGNR TGPGPRSAVP 1200

RRVKQSLKQI RLPLEEIKPE RGVVLNATST RWSESSPILQ GAKRNNLSLP FLTLEMAGGQ 1260

GKISALGKSA AGPLASGKLE KAVLSSAGLS EASGKAEFLP KVRVHREDLL PQKTSNVSCA 1320

HGDLGQEIFL QKTRGPVNLN KVNRPGRTPS KLLGPPMPKE WESLEKSPKS TALRTKDIIS 1380

LPLDRHESNH SIAAKNEGQA ETQREAAWTK QGGPGRLCAP KPPVLRRHQR DISLPTFQPE 1440

EDKMDYDDIF STETKGEDFD IYGEDENQDP RSFQKRTRHY FIAAVEQLWD YGMSESPRAL 1500

RNRAQNGEVP RFKKVVFREF ADGSFTQPSY RGELNKHLGL LGPYIRAEVE DNIMVTFKNQ 1560

ASRPYSFYSS LISYPDDQEQ GAEPRHNFVQ PNETRTYFWK VQHHMAPTED EFDCKAWAYF 1620

SDVDLEKDVH SGLIGPLLIC RANTLNAAHG RQVTVQEFAL FFTIFDETKS WYFTENVERN 1680

CRAPCHLQME DPTLKENYRF HAINGYVMDT LPGLVMAQNQ RIRWYLLSMG SNENIHSIHF 1740

SGHVFSVRKK EEYKMAVYNL YPGVFETVEM LPSKVGIWRI ECLIGEHLQA GMSTTFLVYS 1800

KECQAPLGMA SGRIRDFQIT ASGQYGQWAP KLARLHYSGS INAWSTKDPH SWIKVDLLAP 1860

MIIHGIMTQG ARQKFSSLYI SQFIIMYSLD GRNWQSYRGN STGTLMVFFG NVDASGIKHN 1920

IFNPPIVARY IRLHPTHYSI RSTLRMELMG CDLNSCSMPL GMQNKAISDS QITASSHLSN 1980

IFATWSPSQA RLHLQGRTNA WRPRVSSAEE WLQVDLQKTV KVTGITTQGV KSLLSSMYVK 2040

EFLVSSSQDG RRWTLFLQDG HTKVFQGNQD SSTPVVNALD PPLFTRYLRI HPTSWAQHIA 2100

LRLEVLGCEA QDLY 2114
<212> Type : PRT
<211> Length : 2114
SequenceName : Sequenz 33
SequenceDescription :

Feature
-------

Sequence: Sequenz 33:
<221> FeatureKey : CHAIN
<222> LocationFrom : 1
<222> LocationTo : 2114
Other Information :
CDSJoin : No

Sequence
--------
<213> OrganismName : human
<400> PreSequenceString :
ATRRYYLGAV ELSWDYMQSD LGELPVDARF PPRVPKSFPF NTSVVYKKTL FVEFTDHLFN 60

IAKPRPPWMG LLGPTIQAEV YDTVVITLKN MASHPVSLHA VGVSYWKASE GAEYDDQTSQ 120

REKEDDKVFP GGSHTYVWQV LKENGPMASD PLCLTYSYLS HVDLVKDLNS GLIGALLVCR 180

EGSLAKEKTQ TLHKFILLFA VFDEGKSWHS ETKNSLMQDR DAASARAWPK MHTVNGYVNR 240

SLPGLIGCHR KSVYWHVIGM GTTPEVHSIF LEGHTFLVRN HRQASLEISP ITFLTAQTLL 300

MDLGQFLLFC HISSHQHDGM EAYVKVDSCP EEPQLRMKNN EEAEDYDDDL TDSEMDVVRF 360

DDDNSPSFIQ IRSVAKKHPK TWVHYIAAEE EDWDYAPLVL APDDRSYKSQ YLNNGPQRIG 420

RKYKKVRFMA YTDETFKTRE AIQHESGILG PLLYGEVGDT LLIIFKNQAS RPYNIYPHGI 480

TDVRPLYSRR LPKGVKHLKD FPILPGEIFK YKWTVTVEDG PTKSDPRCLT RYYSSFVNME 540

RDLASGLIGP LLICYKESVD QRGNQIMSDK RNVILFSVFD ENRSWYLTEN IQRFLPNPAG 600

VQLEDPEFQA SNIMHSINGY VFDSLQLSVC LHEVAYWYIL SIGAQTDFLS VFFSGYTFKH 660

KMVYEDTLTL FPFSGETVFM SMENPGLWIL GCHNSDFRNR GMTALLKVSS CDKNTGDYYE 720

DSYEDISAYL LSKNNAIEPR SFSQNSRHRS TRQKQFNATT IPENDIEKTD PWFAHRTPMP 780

KIQNVSSSDL LMLLRQSPTP HGLSLSDLQE AKYETFSDDP SPGAIDSNNS LSEMTHFRPQ 840

LHHSGDMVFT PESGLQLRLN EKLGTTAATE LKKLDFKVSS TSNNLISTIP SDNLAAGTDN 900

TSSLGPPSMP VHYDSQLDTT LFGKKSSPLT ESGGPLSLSE ENNDSKLLES GLMNSQESSW 960

GKNVSSTESG RLFKGKRAHG PALLTKDNAL FKVSISLLKT NKTSNNSATN RKTHIDGPSL 1020

LIENSPSVWQ NILESDTEFK KVTPLIHDRM LMDKNATALR LNHMSNKTTS SKNMEMVQQK 1080

KEGPIPPDAQ NPDMSFFKML FLPESARWIQ RTHGKNSLNS GQGPSPKQLV SLGPEKSVEG 1140

QNFLSEKNKV VVGKGEFTKD VGLKEMVFPS SRNLFLTNLD NLHENNTHNQ EKKIQEEIEK 1200

KETLIQENVV LPQIHTVTGT KNFMKNLFLL STRQNVEGSY DGAYAPVLQD FRSLNDSTNR 1260

TKKHTAHFSK KGEEENLEGL GNQTKQIVEK YACTTRISPN TSQQNFVTQR SKRALKQFRL 1320

PLEETELEKR IIVDDTSTQW SKNMKHLTPS TLTQIDYNEK EKGAITQSPL SDCLTRSHSI 1380

PQANRSPLPI AKVSSFPSIR PIYLTRVLFQ DNSSHLPAAS YRKKDSGVQE SSHFLQGAKK 1440

NNLSLAILTL EMTGDQREVG SLGTSATNSV TYKKVENTVL PKPDLPKTSG KVELLPKVHI 1500

YQKDLFPTET SNGSPGHLDL VEGSLLQGTE GAIKWNEANR PGKVPFLRVA TESSAKTPSK 1560

LLDPLAWDNH YGTQIPKEEW KSQEKSPEKT AFKKKDTILS LNACESNHAI AAINEGQNKP 1620

EIEVTWAKQG RTERLCSQNP PVLKRHQREI TRTTLQSDQE EIDYDDTISV EMKKEDFDIY 1680

DEDENQSPRS FQKKTRHYFI AAVERLWDYG MSSSPHVLRN RAQSGSVPQF KKVVFQEFTD 1740

GSFTQPLYRG ELNEHLGLLG PYIRAEVEDN IMVTFRNQAS RPYSFYSSLI SYEEDQRQGA 1800

EPRKNFVKPN ETKTYFWKVQ HHMAPTKDEF DCKAWAYFSD VDLEKDVHSG LIGPLLVCHT 1860

NTLNPAHGRQ VTVQEFALFF TIFDETKSWY FTENMERNCR APCNIQMEDP TFKENYRFHA 1920

INGYIMDTLP GLVMAQDQRI RWYLLSMGSN ENIHSIHFSG HVFTVRKKEE YKMALYNLYP 1980

GVFETVEMLP SKAGIWRVEC LIGEHLHAGM STLFLVYSNK CQTPLGMASG HIRDFQITAS 2040

GQYGQWAPKL ARLHYSGSIN AWSTKEPFSW IKVDLLAPMI IHGIKTQGAR QKFSSLYISQ 2100

FIIMYSLDGK KWQTYRGNST GTLMVFFGNV DSSGIKHNIF NPPIIARYIR LHPTHYSIRS 2160

TLRMELMGCD LNSCSMPLGM ESKAISDAQI TASSYFTNMF ATWSPSKARL HLQGRSNAWR 2220

PQVNNPKEWL QVDFQKTMKV TGVTTQGVKS LLTSMYVKEF LISSSQDGHQ WTLFFQNGKV 2280

KVFQGNQDSF TPVVNSLDPP LLTRYLRIHP QSWVHQIALR MEVLGCEAQD LY 2332

<212> Type : PRT
<211> Length : 2332
SequenceName : Sequenz 34
SequenceDescription :

Feature
-------
Sequence: Sequenz 34:
<221> FeatureKey : CHAIN
<222> LocationFrom : 1
<222> LocationTo : 2332
Other Information :
CDSJoin : No

SEQUENCE LISTING

<110> Aventis Behring GmbH

<120> Modified cDNA Factor VIII and its Derivatives

<130> A66

<160> 34

<170> PatentIn version 3.1

<210> 1
<211> 34
<212> DNA
<213> Artificial

<400> 1
ggaaccatcg ccagaagtcc ttggaaatct cgcc 34

<210> 2
<211> 34
<212> DNA
<213> Artificial

<400> 2
ggcgagattt ccaaggactt ctggcgatgg ttcc 34

<210> 3
<211> 32
<212> DNA
<213> Artificial

<400> 3
cccaccaaca tggtggcatg gaagcttatg tc 32

<210> 4

Sequence Listing korrekt.txt
<211>  32

<212>  DNA

<213>  Artificial


<400>  4
gacataagct tccatgccac catgttggtg gg                                    32

<210>  5

<211>  47

<212>  DNA

<213>  Artificial


<400>  5
cagaggaacc ccaactacga cgtaaaaata atgaagaagc ggaagac                    47

<210>  6

<211>  47

<212>  DNA

<213>  Artificial


<400>  6
gtcttccgct tcttcattat ttttacgtcg tagttggggt tcctctg                    47

<210>  7

<211>  42

<212>  DNA

<213>  Artificial


<400>  7
cccaactacg aatgaaaaat gatgaagaag cggaagacta tg                         42

<210>  8

<211>  42

<212>  DNA

<213>  Artificial


<400>  8
catagtcttc cgcttcttca tcatttttca ttcgtagttg gg                         42

Sequence Listing korrekt.txt
<210> 9

<211> 40

<212> DNA

<213> Artificial


<400> 9
gaagaagcgg aagactatga tgataatctt actgattctg 40

<210> 10

<211> 40

<212> DNA

<213> Artificial


<400> 10
cagaatcagt aagattatca tcatagtctt ccgcttcttc 40

<210> 11

<211> 38

<212> DNA

<213> Artificial


<400> 11
ggtcaggttt gatgatgacg actctccttc ctttatcc 38

<210> 12

<211> 38

<212> DNA

<213> Artificial


<400> 12
ggataaagga aggagagtcg tcatcatcaa acctgacc 38

<210> 13

<211> 38

<212> DNA

<213> Artificial


<400> 13
cccttagtcc tcgccccctc tgacagaagt tataaaag 38

Sequence Listing korrekt.txt

<210>  14

<211>  38

<212>  DNA

<213>  Artificial

<400>  14
cttttataac ttctgtcaga gggggcgagg actaaggg                              38

<210>  15

<211>  41

<212>  DNA

<213>  Artificial

<400>  15
gtccgattta tggcatacac agatgttacc tttaagactc g                          41

<210>  16

<211>  41

<212>  DNA

<213>  Artificial

<400>  16
cgagtcttaa aggtaacatc tgtgtatgcc ataaatcgga c                          41

<210>  17

<211>  37

<212>  DNA

<213>  Artificial

<400>  17
cctttaagac tcgtaaagct attcagcatg aatcagg                               37

<210>  18

<211>  37

<212>  DNA

<213>  Artificial

Sequence Listing korrekt.txt
<400> 18
cctgattcat gctgaatagc tttacgagtc ttaaagg 37

<210> 19

<211> 46

<212> DNA

<213> Artificial

<400> 19
cacactgttg attatattta agaataaagc aagcagacca tataac 46

<210> 20

<211> 46

<212> DNA

<213> Artificial

<400> 20
gttatatggt ctgcttgctt tattcttaaa tataatcaac agtgtg 46

<210> 21

<211> 40

<212> DNA

<213> Artificial

<400> 21
ccctcacgga atcactgatg tctctccttt gtattcaagg 40

<210> 22

<211> 40

<212> DNA

<213> Artificial

<400> 22
ccttgaatac aaaggagaga catcagtgat tccgtgaggg 40

<210> 23

<211> 38

<212> DNA

<213> Artificial

Sequence Listing korrekt.txt

<400> 23
gatgtccgtc ctttgtattc agggagatta ccaaaagg 38

<210> 24

<211> 38

<212> DNA

<213> Artificial

<400> 24
ccttttggta atctccctga atacaaagga cggacatc 38

<210> 25

<211> 36

<212> DNA

<213> Artificial

<400> 25
ctgtatttga tgagaaccaa agctggtacc tcacag 36

<210> 26

<211> 36

<212> DNA

<213> Artificial

<400> 26
ctgtgaggta ccagctttgg ttctcatcaa atacag 36

<210> 27

<211> 30

<212> DNA

<213> Artificial

<400> 27
ctccccaatc cagatggagt gcagcttgag 30

<210> 28

<211> 30

<212> DNA

Sequence Listing korrekt.txt
<213>  Artificial

<400>  28
ctcaagctgc actccatctg gattggggag                                        30

<210>  29

<211>  32

<212>  DNA

<213>  Artificial

<400>  29
cagctggagt gcagcttcag gatccagagt tc                                     32

<210>  30

<211>  32

<212>  DNA

<213>  Artificial

<400>  30
gaactctgga tcctgaagct gcactccagc tg                                     32

<210>  31

<211>  49

<212>  DNA

<213>  Artificial

<400>  31
cgatggtatc tgctcagcat gaagagcaat gaaaacatcc attctattc                   49

<210>  32

<211>  49

<212>  DNA

<213>  Artificial

<400>  32
gaatagaatg gatgttttca ttgctcttca tgctgagcag ataccatcg                   49

<210>  33

<211>  2114

Sequence Listing korrekt.txt
<212>  PRT

<213>  Porcine

<400>  33

Ala Ile Arg Arg Tyr Tyr Leu Gly Ala Val Glu Leu Ser Trp Asp Tyr
1               5                   10                  15

Arg Gln Ser Glu Leu Leu Arg Glu Leu His Val Asp Thr Arg Phe Pro
            20                  25                  30

Ala Thr Ala Pro Gly Ala Leu Pro Leu Gly Pro Ser Val Leu Tyr Lys
        35                  40                  45

Lys Thr Val Phe Val Glu Phe Thr Asp Gln Leu Phe Ser Val Ala Arg
    50                  55                  60

Pro Arg Pro Pro Trp Met Gly Leu Leu Gly Pro Thr Ile Gln Ala Glu
65                  70                  75                  80

Val Tyr Asp Thr Val Val Val Thr Leu Lys Asn Met Ala Ser His Pro
                85                  90                  95

Val Ser Leu His Ala Val Gly Val Ser Phe Trp Lys Ser Ser Glu Gly
            100                 105                 110

Ala Glu Tyr Glu Asp His Thr Ser Gln Arg Glu Lys Glu Asp Asp Lys
        115                 120                 125

Val Leu Pro Gly Lys Ser Gln Thr Tyr Val Trp Gln Val Leu Lys Glu
    130                 135                 140

Asn Gly Pro Thr Ala Ser Asp Pro Pro Cys Leu Thr Tyr Ser Tyr Leu
145                 150                 155                 160

Ser His Val Asp Leu Val Lys Asp Leu Asn Ser Gly Leu Ile Gly Ala
                165                 170                 175

Leu Leu Val Cys Arg Glu Gly Ser Leu Thr Arg Glu Arg Thr Gln Asn
            180                 185                 190

Leu His Glu Phe Val Leu Leu Phe Ala Val Phe Asp Glu Gly Lys Ser
        195                 200                 205

Trp His Ser Ala Arg Asn Asp Ser Trp Thr Arg Ala Met Asp Pro Ala
    210                 215                 220

Pro Ala Arg Ala Gln Pro Ala Met His Thr Val Asn Gly Tyr Val Asn
225                 230                 235                 240

Sequence Listing korrekt.txt
Arg Ser Leu Pro Gly Leu Ile Gly Cys His Lys Lys Ser Val Tyr Trp
                245                 250                 255

His Val Ile Gly Met Gly Thr Ser Pro Glu Val His Ser Ile Phe Leu
            260                 265                 270

Glu Gly His Thr Phe Leu Val Arg His His Arg Gln Ala Ser Leu Glu
        275                 280                 285

Ile Ser Pro Leu Thr Phe Leu Thr Ala Gln Thr Phe Leu Met Asp Leu
    290                 295                 300

Gly Gln Phe Leu Leu Phe Cys His Ile Ser Ser His His His Gly Gly
305                 310                 315                 320

Met Glu Ala His Val Arg Val Glu Ser Cys Ala Glu Glu Pro Gln Leu
                325                 330                 335

Arg Arg Lys Ala Asp Glu Glu Glu Asp Tyr Asp Asp Asn Leu Tyr Asp
            340                 345                 350

Ser Asp Met Asp Val Val Arg Leu Asp Gly Asp Asp Val Ser Pro Phe
        355                 360                 365

Ile Gln Ile Arg Ser Val Ala Lys Lys His Pro Lys Thr Trp Val His
    370                 375                 380

Tyr Ile Ser Ala Glu Glu Glu Asp Trp Asp Tyr Ala Pro Ala Val Pro
385                 390                 395                 400

Ser Pro Ser Asp Arg Ser Tyr Lys Ser Leu Tyr Leu Asn Ser Gly Pro
                405                 410                 415

Gln Arg Ile Gly Arg Lys Tyr Lys Lys Ala Arg Phe Val Ala Tyr Thr
            420                 425                 430

Asp Val Thr Phe Lys Thr Arg Lys Ala Ile Pro Tyr Glu Ser Gly Ile
        435                 440                 445

Leu Gly Pro Leu Leu Tyr Gly Glu Val Gly Asp Thr Leu Leu Ile Ile
    450                 455                 460

Phe Lys Asn Lys Ala Ser Arg Pro Tyr Asn Ile Tyr Pro His Gly Ile
465                 470                 475                 480

Thr Asp Val Ser Ala Leu His Pro Gly Arg Leu Leu Lys Gly Trp Lys
                485                 490                 495

His Leu Lys Asp Met Pro Ile Leu Pro Gly Glu Thr Phe Lys Tyr Lys
            500                 505                 510

Sequence Listing korrekt.txt
Trp Thr Val Thr Val Glu Asp Gly Pro Thr Lys Ser Asp Pro Arg Cys
        515                 520                 525

Leu Thr Arg Tyr Tyr Ser Ser Ser Ile Asn Leu Glu Lys Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Met Met Ser Asp Lys Arg Asn Val Ile Leu Phe
                565                 570                 575

Ser Val Phe Asp Glu Asn Gln Ser Trp Tyr Leu Ala Glu Asn Ile Gln
            580                 585                 590

Arg Phe Leu Pro Asn Pro Asp Gly Leu Gln Pro Gln Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Val Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Val Leu Gly Cys His Asn Ser Asp Leu Arg Asn Arg Gly Met Thr Ala
    690                 695                 700

Leu Leu Lys Val Tyr Ser Cys Asp Arg Asp Ile Gly Asp Tyr Tyr Asp
705                 710                 715                 720

Asn Thr Tyr Glu Asp Ile Pro Gly Phe Leu Leu Ser Gly Lys Asn Val
                725                 730                 735

Ile Glu Pro Arg Ser Phe Ala Gln Asn Ser Arg Pro Pro Ser Ala Ser
            740                 745                 750

Gln Lys Gln Phe Gln Thr Ile Thr Ser Pro Glu Asp Asp Val Glu Leu
        755                 760                 765

Asp Pro Gln Ser Gly Glu Arg Thr Gln Ala Leu Glu Glu Leu Ser Val
    770                 775                 780

Sequence Listing korrekt.txt
Pro Ser Gly Asp Gly Ser Met Leu Leu Gly Gln Asn Pro Ala Pro His
785                 790                 795                 800

Gly Ser Ser Ser Ser Asp Leu Gln Glu Ala Arg Asn Glu Ala Asp Asp
                805                 810                 815

Tyr Leu Pro Gly Ala Arg Glu Arg Asn Thr Ala Pro Ser Ala Ala Ala
            820                 825                 830

Arg Leu Arg Pro Glu Leu His His Ser Ala Glu Arg Val Leu Thr Pro
        835                 840                 845

Glu Pro Glu Lys Glu Leu Lys Lys Leu Asp Ser Lys Met Ser Ser Ser
    850                 855                 860

Ser Asp Leu Leu Lys Thr Ser Pro Thr Ile Pro Ser Asp Thr Leu Ser
865                 870                 875                 880

Ala Glu Thr Glu Arg Thr His Ser Leu Gly Pro Pro His Pro Gln Val
                885                 890                 895

Asn Phe Arg Ser Gln Leu Gly Ala Ile Val Leu Gly Lys Asn Ser Ser
            900                 905                 910

His Phe Ile Gly Ala Gly Val Pro Leu Gly Ser Thr Glu Glu Asp His
        915                 920                 925

Glu Ser Ser Leu Gly Glu Asn Val Ser Pro Val Glu Ser Asp Gly Ile
    930                 935                 940

Phe Glu Lys Glu Arg Ala His Gly Pro Ala Ser Leu Thr Lys Asp Asp
945                 950                 955                 960

Val Leu Phe Lys Val Asn Ile Ser Leu Val Lys Thr Asn Lys Ala Arg
                965                 970                 975

Val Tyr Leu Lys Thr Asn Arg Lys Ile His Ile Asp Asp Ala Ala Leu
            980                 985                 990

Leu Thr Glu Asn Arg Ala Ser Ala  Thr Phe Met Asp Lys  Asn Thr Thr
        995                 1000                 1005

Ala Ser  Gly Leu Asn His Val  Ser Asn Trp Ile Lys  Gly Pro Leu
    1010                 1015                 1020

Gly Lys  Asn Pro Leu Ser Ser  Glu Arg Gly Pro Ser  Pro Glu Leu
    1025                 1030                 1035

Leu Thr  Ser Ser Gly Ser Gly  Lys Ser Val Lys Gly  Gln Ser Ser
    1040                 1045                 1050

Sequence Listing korrekt.txt
Gly Gln Gly Arg Ile Arg Val Ala Val Glu Glu Glu Glu Leu Ser
1055 1060 1065

Lys Gly Lys Glu Met Met Leu Pro Asn Ser Glu Leu Thr Phe Leu
1070 1075 1080

Thr Asn Ser Ala Asp Val Gln Gly Asn Asp Thr His Ser Gln Gly
1085 1090 1095

Lys Lys Ser Arg Glu Glu Met Glu Arg Arg Glu Lys Leu Val Gln
1100 1105 1110

Glu Lys Val Asp Leu Pro Gln Val Tyr Thr Ala Thr Gly Thr Lys
1115 1120 1125

Asn Phe Leu Arg Asn Ile Phe His Gln Ser Thr Glu Pro Ser Val
1130 1135 1140

Glu Gly Phe Asp Gly Gly Ser His Ala Pro Val Pro Gln Asp Ser
1145 1150 1155

Arg Ser Leu Asn Asp Ser Ala Glu Arg Ala Glu Thr His Ile Ala
1160 1165 1170

His Phe Ser Ala Ile Arg Glu Glu Ala Pro Leu Glu Ala Pro Gly
1175 1180 1185

Asn Arg Thr Gly Pro Gly Pro Arg Ser Ala Val Pro Arg Arg Val
1190 1195 1200

Lys Gln Ser Leu Lys Gln Ile Arg Leu Pro Leu Glu Glu Ile Lys
1205 1210 1215

Pro Glu Arg Gly Val Val Leu Asn Ala Thr Ser Thr Arg Trp Ser
1220 1225 1230

Glu Ser Ser Pro Ile Leu Gln Gly Ala Lys Arg Asn Asn Leu Ser
1235 1240 1245

Leu Pro Phe Leu Thr Leu Glu Met Ala Gly Gly Gln Gly Lys Ile
1250 1255 1260

Ser Ala Leu Gly Lys Ser Ala Ala Gly Pro Leu Ala Ser Gly Lys
1265 1270 1275

Leu Glu Lys Ala Val Leu Ser Ser Ala Gly Leu Ser Glu Ala Ser
1280 1285 1290

Gly Lys Ala Glu Phe Leu Pro Lys Val Arg Val His Arg Glu Asp
1295 1300 1305

Sequence Listing korrekt.txt
Leu Leu Pro Gln Lys Thr Ser Asn Val Ser Cys Ala His Gly Asp
1310 1315 1320

Leu Gly Gln Glu Ile Phe Leu Gln Lys Thr Arg Gly Pro Val Asn
1325 1330 1335

Leu Asn Lys Val Asn Arg Pro Gly Arg Thr Pro Ser Lys Leu Leu
1340 1345 1350

Gly Pro Pro Met Pro Lys Glu Trp Glu Ser Leu Glu Lys Ser Pro
1355 1360 1365

Lys Ser Thr Ala Leu Arg Thr Lys Asp Ile Ile Ser Leu Pro Leu
1370 1375 1380

Asp Arg His Glu Ser Asn His Ser Ile Ala Ala Lys Asn Glu Gly
1385 1390 1395

Gln Ala Glu Thr Gln Arg Glu Ala Ala Trp Thr Lys Gln Gly Gly
1400 1405 1410

Pro Gly Arg Leu Cys Ala Pro Lys Pro Pro Val Leu Arg Arg His
1415 1420 1425

Gln Arg Asp Ile Ser Leu Pro Thr Phe Gln Pro Glu Glu Asp Lys
1430 1435 1440

Met Asp Tyr Asp Asp Ile Phe Ser Thr Glu Thr Lys Gly Glu Asp
1445 1450 1455

Phe Asp Ile Tyr Gly Glu Asp Glu Asn Gln Asp Pro Arg Ser Phe
1460 1465 1470

Gln Lys Arg Thr Arg His Tyr Phe Ile Ala Ala Val Glu Gln Leu
1475 1480 1485

Trp Asp Tyr Gly Met Ser Glu Ser Pro Arg Ala Leu Arg Asn Arg
1490 1495 1500

Ala Gln Asn Gly Glu Val Pro Arg Phe Lys Lys Val Val Phe Arg
1505 1510 1515

Glu Phe Ala Asp Gly Ser Phe Thr Gln Pro Ser Tyr Arg Gly Glu
1520 1525 1530

Leu Asn Lys His Leu Gly Leu Leu Gly Pro Tyr Ile Arg Ala Glu
1535 1540 1545

Val Glu Asp Asn Ile Met Val Thr Phe Lys Asn Gln Ala Ser Arg
1550 1555 1560

Sequence Listing korrekt.txt
Pro Tyr Ser Phe Tyr Ser Ser Leu Ile Ser Tyr Pro Asp Asp Gln
    1565                1570                1575

Glu Gln Gly Ala Glu Pro Arg His Asn Phe Val Gln Pro Asn Glu
    1580                1585                1590

Thr Arg Thr Tyr Phe Trp Lys Val Gln His His Met Ala Pro Thr
    1595                1600                1605

Glu Asp Glu Phe Asp Cys Lys Ala Trp Ala Tyr Phe Ser Asp Val
    1610                1615                1620

Asp Leu Glu Lys Asp Val His Ser Gly Leu Ile Gly Pro Leu Leu
    1625                1630                1635

Ile Cys Arg Ala Asn Thr Leu Asn Ala Ala His Gly Arg Gln Val
    1640                1645                1650

Thr Val Gln Glu Phe Ala Leu Phe Phe Thr Ile Phe Asp Glu Thr
    1655                1660                1665

Lys Ser Trp Tyr Phe Thr Glu Asn Val Glu Arg Asn Cys Arg Ala
    1670                1675                1680

Pro Cys His Leu Gln Met Glu Asp Pro Thr Leu Lys Glu Asn Tyr
    1685                1690                1695

Arg Phe His Ala Ile Asn Gly Tyr Val Met Asp Thr Leu Pro Gly
    1700                1705                1710

Leu Val Met Ala Gln Asn Gln Arg Ile Arg Trp Tyr Leu Leu Ser
    1715                1720                1725

Met Gly Ser Asn Glu Asn Ile His Ser Ile His Phe Ser Gly His
    1730                1735                1740

Val Phe Ser Val Arg Lys Lys Glu Glu Tyr Lys Met Ala Val Tyr
    1745                1750                1755

Asn Leu Tyr Pro Gly Val Phe Glu Thr Val Glu Met Leu Pro Ser
    1760                1765                1770

Lys Val Gly Ile Trp Arg Ile Glu Cys Leu Ile Gly Glu His Leu
    1775                1780                1785

Gln Ala Gly Met Ser Thr Thr Phe Leu Val Tyr Ser Lys Glu Cys
    1790                1795                1800

Gln Ala Pro Leu Gly Met Ala Ser Gly Arg Ile Arg Asp Phe Gln
    1805                1810                1815

Sequence Listing korrekt.txt
Ile Thr Ala Ser Gly Gln Tyr Gly Gln Trp Ala Pro Lys Leu Ala
    1820                    1825                1830

Arg Leu His Tyr Ser Gly Ser Ile Asn Ala Trp Ser Thr Lys Asp
    1835                    1840                1845

Pro His Ser Trp Ile Lys Val Asp Leu Leu Ala Pro Met Ile Ile
    1850                    1855                1860

His Gly Ile Met Thr Gln Gly Ala Arg Gln Lys Phe Ser Ser Leu
    1865                    1870                1875

Tyr Ile Ser Gln Phe Ile Ile Met Tyr Ser Leu Asp Gly Arg Asn
    1880                    1885                1890

Trp Gln Ser Tyr Arg Gly Asn Ser Thr Gly Thr Leu Met Val Phe
    1895                    1900                1905

Phe Gly Asn Val Asp Ala Ser Gly Ile Lys His Asn Ile Phe Asn
    1910                    1915                1920

Pro Pro Ile Val Ala Arg Tyr Ile Arg Leu His Pro Thr His Tyr
    1925                    1930                1935

Ser Ile Arg Ser Thr Leu Arg Met Glu Leu Met Gly Cys Asp Leu
    1940                    1945                1950

Asn Ser Cys Ser Met Pro Leu Gly Met Gln Asn Lys Ala Ile Ser
    1955                    1960                1965

Asp Ser Gln Ile Thr Ala Ser Ser His Leu Ser Asn Ile Phe Ala
    1970                    1975                1980

Thr Trp Ser Pro Ser Gln Ala Arg Leu His Leu Gln Gly Arg Thr
    1985                    1990                1995

Asn Ala Trp Arg Pro Arg Val Ser Ser Ala Glu Glu Trp Leu Gln
    2000                    2005                2010

Val Asp Leu Gln Lys Thr Val Lys Val Thr Gly Ile Thr Thr Gln
    2015                    2020                2025

Gly Val Lys Ser Leu Leu Ser Ser Met Tyr Val Lys Glu Phe Leu
    2030                    2035                2040

Val Ser Ser Ser Gln Asp Gly Arg Arg Trp Thr Leu Phe Leu Gln
    2045                    2050                2055

Asp Gly His Thr Lys Val Phe Gln Gly Asn Gln Asp Ser Ser Thr
    2060                    2065                2070

Sequence Listing korrekt.txt
Pro Val Val Asn Ala Leu Asp Pro Pro Leu Phe Thr Arg Tyr Leu
2075 2080 2085

Arg Ile His Pro Thr Ser Trp Ala Gln His Ile Ala Leu Arg Leu
2090 2095 2100

Glu Val Leu Gly Cys Glu Ala Gln Asp Leu Tyr
2105 2110

<210> 34

<211> 2332

<212> PRT

<213> Homo sapiens

<400> 34

Ala Thr Arg Arg Tyr Tyr Leu Gly Ala Val Glu Leu Ser Trp Asp Tyr
1               5                   10                  15

Met Gln Ser Asp Leu Gly Glu Leu Pro Val Asp Ala Arg Phe Pro Pro
            20                  25                  30

Arg Val Pro Lys Ser Phe Pro Phe Asn Thr Ser Val Val Tyr Lys Lys
        35                  40                  45

Thr Leu Phe Val Glu Phe Thr Asp His Leu Phe Asn Ile Ala Lys Pro
    50                  55                  60

Arg Pro Pro Trp Met Gly Leu Leu Gly Pro Thr Ile Gln Ala Glu Val
65                  70                  75                  80

Tyr Asp Thr Val Val Ile Thr Leu Lys Asn Met Ala Ser His Pro Val
                85                  90                  95

Ser Leu His Ala Val Gly Val Ser Tyr Trp Lys Ala Ser Glu Gly Ala
            100                 105                 110

Glu Tyr Asp Asp Gln Thr Ser Gln Arg Glu Lys Glu Asp Asp Lys Val
        115                 120                 125

Phe Pro Gly Gly Ser His Thr Tyr Val Trp Gln Val Leu Lys Glu Asn
    130                 135                 140

Gly Pro Met Ala Ser Asp Pro Leu Cys Leu Thr Tyr Ser Tyr Leu Ser
145                 150                 155                 160

His Val Asp Leu Val Lys Asp Leu Asn Ser Gly Leu Ile Gly Ala Leu
                165                 170                 175

Sequence Listing korrekt.txt
Leu Val Cys Arg Glu Gly Ser Leu Ala Lys Glu Lys Thr Gln Thr Leu
            180                 185                 190

His Lys Phe Ile Leu Leu Phe Ala Val Phe Asp Glu Gly Lys Ser Trp
        195                 200                 205

His Ser Glu Thr Lys Asn Ser Leu Met Gln Asp Arg Asp Ala Ala Ser
    210                 215                 220

Ala Arg Ala Trp Pro Lys Met His Thr Val Asn Gly Tyr Val Asn Arg
225                 230                 235                 240

Ser Leu Pro Gly Leu Ile Gly Cys His Arg Lys Ser Val Tyr Trp His
                245                 250                 255

Val Ile Gly Met Gly Thr Thr Pro Glu Val His Ser Ile Phe Leu Glu
            260                 265                 270

Gly His Thr Phe Leu Val Arg Asn His Arg Gln Ala Ser Leu Glu Ile
        275                 280                 285

Ser Pro Ile Thr Phe Leu Thr Ala Gln Thr Leu Leu Met Asp Leu Gly
    290                 295                 300

Gln Phe Leu Leu Phe Cys His Ile Ser Ser His Gln His Asp Gly Met
305                 310                 315                 320

Glu Ala Tyr Val Lys Val Asp Ser Cys Pro Glu Glu Pro Gln Leu Arg
                325                 330                 335

Met Lys Asn Asn Glu Glu Ala Glu Asp Tyr Asp Asp Asp Leu Thr Asp
            340                 345                 350

Ser Glu Met Asp Val Val Arg Phe Asp Asp Asp Asn Ser Pro Ser Phe
        355                 360                 365

Ile Gln Ile Arg Ser Val Ala Lys Lys His Pro Lys Thr Trp Val His
    370                 375                 380

Tyr Ile Ala Ala Glu Glu Glu Asp Trp Asp Tyr Ala Pro Leu Val Leu
385                 390                 395                 400

Ala Pro Asp Asp Arg Ser Tyr Lys Ser Gln Tyr Leu Asn Asn Gly Pro
                405                 410                 415

Gln Arg Ile Gly Arg Lys Tyr Lys Lys Val Arg Phe Met Ala Tyr Thr
            420                 425                 430

Asp Glu Thr Phe Lys Thr Arg Glu Ala Ile Gln His Glu Ser Gly Ile
        435                 440                 445

Sequence Listing korrekt.txt
Leu Gly Pro Leu Leu Tyr Gly Glu Val Gly Asp Thr Leu Leu Ile Ile
    450                 455                 460

Phe Lys Asn Gln Ala Ser Arg Pro Tyr Asn Ile Tyr Pro His Gly Ile
465                 470                 475                 480

Thr Asp Val Arg Pro Leu Tyr Ser Arg Arg Leu Pro Lys Gly Val Lys
                485                 490                 495

His Leu Lys Asp Phe Pro Ile Leu Pro Gly Glu Ile Phe Lys Tyr Lys
            500                 505                 510

Trp Thr Val Thr Val Glu Asp Gly Pro Thr Lys Ser Asp Pro Arg Cys
        515                 520                 525

Leu Thr Arg Tyr Tyr Ser Ser Phe Val Asn Met Glu Arg Asp Leu Ala
    530                 535                 540

Ser Gly Leu Ile Gly Pro Leu Leu Ile Cys Tyr Lys Glu Ser Val Asp
545                 550                 555                 560

Gln Arg Gly Asn Gln Ile Met Ser Asp Lys Arg Asn Val Ile Leu Phe
                565                 570                 575

Ser Val Phe Asp Glu Asn Arg Ser Trp Tyr Leu Thr Glu Asn Ile Gln
            580                 585                 590

Arg Phe Leu Pro Asn Pro Ala Gly Val Gln Leu Glu Asp Pro Glu Phe
        595                 600                 605

Gln Ala Ser Asn Ile Met His Ser Ile Asn Gly Tyr Val Phe Asp Ser
    610                 615                 620

Leu Gln Leu Ser Val Cys Leu His Glu Val Ala Tyr Trp Tyr Ile Leu
625                 630                 635                 640

Ser Ile Gly Ala Gln Thr Asp Phe Leu Ser Val Phe Phe Ser Gly Tyr
                645                 650                 655

Thr Phe Lys His Lys Met Val Tyr Glu Asp Thr Leu Thr Leu Phe Pro
            660                 665                 670

Phe Ser Gly Glu Thr Val Phe Met Ser Met Glu Asn Pro Gly Leu Trp
        675                 680                 685

Ile Leu Gly Cys His Asn Ser Asp Phe Arg Asn Arg Gly Met Thr Ala
    690                 695                 700

Leu Leu Lys Val Ser Ser Cys Asp Lys Asn Thr Gly Asp Tyr Tyr Glu
705                 710                 715                 720

Sequence Listing korrekt.txt
Asp Ser Tyr Glu Asp Ile Ser Ala Tyr Leu Leu Ser Lys Asn Asn Ala
                725                 730                 735

Ile Glu Pro Arg Ser Phe Ser Gln Asn Ser Arg His Arg Ser Thr Arg
            740                 745                 750

Gln Lys Gln Phe Asn Ala Thr Thr Ile Pro Glu Asn Asp Ile Glu Lys
        755                 760                 765

Thr Asp Pro Trp Phe Ala His Arg Thr Pro Met Pro Lys Ile Gln Asn
    770                 775                 780

Val Ser Ser Ser Asp Leu Leu Met Leu Leu Arg Gln Ser Pro Thr Pro
785                 790                 795                 800

His Gly Leu Ser Leu Ser Asp Leu Gln Glu Ala Lys Tyr Glu Thr Phe
                805                 810                 815

Ser Asp Asp Pro Ser Pro Gly Ala Ile Asp Ser Asn Asn Ser Leu Ser
            820                 825                 830

Glu Met Thr His Phe Arg Pro Gln Leu His His Ser Gly Asp Met Val
        835                 840                 845

Phe Thr Pro Glu Ser Gly Leu Gln Leu Arg Leu Asn Glu Lys Leu Gly
    850                 855                 860

Thr Thr Ala Ala Thr Glu Leu Lys Lys Leu Asp Phe Lys Val Ser Ser
865                 870                 875                 880

Thr Ser Asn Asn Leu Ile Ser Thr Ile Pro Ser Asp Asn Leu Ala Ala
                885                 890                 895

Gly Thr Asp Asn Thr Ser Ser Leu Gly Pro Pro Ser Met Pro Val His
            900                 905                 910

Tyr Asp Ser Gln Leu Asp Thr Thr Leu Phe Gly Lys Lys Ser Ser Pro
        915                 920                 925

Leu Thr Glu Ser Gly Gly Pro Leu Ser Leu Ser Glu Glu Asn Asn Asp
    930                 935                 940

Ser Lys Leu Leu Glu Ser Gly Leu Met Asn Ser Gln Glu Ser Ser Trp
945                 950                 955                 960

Gly Lys Asn Val Ser Ser Thr Glu Ser Gly Arg Leu Phe Lys Gly Lys
                965                 970                 975

Arg Ala His Gly Pro Ala Leu Leu Thr Lys Asp Asn Ala Leu Phe Lys
            980                 985                 990

Sequence Listing korrekt.txt
Val Ser Ile Ser Leu Leu Lys Thr Asn Lys Thr Ser Asn Asn Ser Ala
995 1000 1005

Thr Asn Arg Lys Thr His Ile Asp Gly Pro Ser Leu Leu Ile Glu
1010 1015 1020

Asn Ser Pro Ser Val Trp Gln Asn Ile Leu Glu Ser Asp Thr Glu
1025 1030 1035

Phe Lys Lys Val Thr Pro Leu Ile His Asp Arg Met Leu Met Asp
1040 1045 1050

Lys Asn Ala Thr Ala Leu Arg Leu Asn His Met Ser Asn Lys Thr
1055 1060 1065

Thr Ser Ser Lys Asn Met Glu Met Val Gln Gln Lys Lys Glu Gly
1070 1075 1080

Pro Ile Pro Pro Asp Ala Gln Asn Pro Asp Met Ser Phe Phe Lys
1085 1090 1095

Met Leu Phe Leu Pro Glu Ser Ala Arg Trp Ile Gln Arg Thr His
1100 1105 1110

Gly Lys Asn Ser Leu Asn Ser Gly Gln Gly Pro Ser Pro Lys Gln
1115 1120 1125

Leu Val Ser Leu Gly Pro Glu Lys Ser Val Glu Gly Gln Asn Phe
1130 1135 1140

Leu Ser Glu Lys Asn Lys Val Val Val Gly Lys Gly Glu Phe Thr
1145 1150 1155

Lys Asp Val Gly Leu Lys Glu Met Val Phe Pro Ser Ser Arg Asn
1160 1165 1170

Leu Phe Leu Thr Asn Leu Asp Asn Leu His Glu Asn Asn Thr His
1175 1180 1185

Asn Gln Glu Lys Lys Ile Gln Glu Glu Ile Glu Lys Lys Glu Thr
1190 1195 1200

Leu Ile Gln Glu Asn Val Val Leu Pro Gln Ile His Thr Val Thr
1205 1210 1215

Gly Thr Lys Asn Phe Met Lys Asn Leu Phe Leu Leu Ser Thr Arg
1220 1225 1230

Gln Asn Val Glu Gly Ser Tyr Asp Gly Ala Tyr Ala Pro Val Leu
1235 1240 1245

Sequence Listing korrekt.txt
Gln Asp Phe Arg Ser Leu Asn Asp Ser Thr Asn Arg Thr Lys Lys
1250 1255 1260

His Thr Ala His Phe Ser Lys Lys Gly Glu Glu Glu Asn Leu Glu
1265 1270 1275

Gly Leu Gly Asn Gln Thr Lys Gln Ile Val Glu Lys Tyr Ala Cys
1280 1285 1290

Thr Thr Arg Ile Ser Pro Asn Thr Ser Gln Gln Asn Phe Val Thr
1295 1300 1305

Gln Arg Ser Lys Arg Ala Leu Lys Gln Phe Arg Leu Pro Leu Glu
1310 1315 1320

Glu Thr Glu Leu Glu Lys Arg Ile Ile Val Asp Asp Thr Ser Thr
1325 1330 1335

Gln Trp Ser Lys Asn Met Lys His Leu Thr Pro Ser Thr Leu Thr
1340 1345 1350

Gln Ile Asp Tyr Asn Glu Lys Glu Lys Gly Ala Ile Thr Gln Ser
1355 1360 1365

Pro Leu Ser Asp Cys Leu Thr Arg Ser His Ser Ile Pro Gln Ala
1370 1375 1380

Asn Arg Ser Pro Leu Pro Ile Ala Lys Val Ser Ser Phe Pro Ser
1385 1390 1395

Ile Arg Pro Ile Tyr Leu Thr Arg Val Leu Phe Gln Asp Asn Ser
1400 1405 1410

Ser His Leu Pro Ala Ala Ser Tyr Arg Lys Lys Asp Ser Gly Val
1415 1420 1425

Gln Glu Ser Ser His Phe Leu Gln Gly Ala Lys Lys Asn Asn Leu
1430 1435 1440

Ser Leu Ala Ile Leu Thr Leu Glu Met Thr Gly Asp Gln Arg Glu
1445 1450 1455

Val Gly Ser Leu Gly Thr Ser Ala Thr Asn Ser Val Thr Tyr Lys
1460 1465 1470

Lys Val Glu Asn Thr Val Leu Pro Lys Pro Asp Leu Pro Lys Thr
1475 1480 1485

Ser Gly Lys Val Glu Leu Leu Pro Lys Val His Ile Tyr Gln Lys
1490 1495 1500

Sequence Listing korrekt.txt
Asp Leu Phe Pro Thr Glu Thr Ser Asn Gly Ser Pro Gly His Leu
1505 1510 1515

Asp Leu Val Glu Gly Ser Leu Leu Gln Gly Thr Glu Gly Ala Ile
1520 1525 1530

Lys Trp Asn Glu Ala Asn Arg Pro Gly Lys Val Pro Phe Leu Arg
1535 1540 1545

Val Ala Thr Glu Ser Ser Ala Lys Thr Pro Ser Lys Leu Leu Asp
1550 1555 1560

Pro Leu Ala Trp Asp Asn His Tyr Gly Thr Gln Ile Pro Lys Glu
1565 1570 1575

Glu Trp Lys Ser Gln Glu Lys Ser Pro Glu Lys Thr Ala Phe Lys
1580 1585 1590

Lys Lys Asp Thr Ile Leu Ser Leu Asn Ala Cys Glu Ser Asn His
1595 1600 1605

Ala Ile Ala Ala Ile Asn Glu Gly Gln Asn Lys Pro Glu Ile Glu
1610 1615 1620

Val Thr Trp Ala Lys Gln Gly Arg Thr Glu Arg Leu Cys Ser Gln
1625 1630 1635

Asn Pro Pro Val Leu Lys Arg His Gln Arg Glu Ile Thr Arg Thr
1640 1645 1650

Thr Leu Gln Ser Asp Gln Glu Glu Ile Asp Tyr Asp Asp Thr Ile
1655 1660 1665

Ser Val Glu Met Lys Lys Glu Asp Phe Asp Ile Tyr Asp Glu Asp
1670 1675 1680

Glu Asn Gln Ser Pro Arg Ser Phe Gln Lys Lys Thr Arg His Tyr
1685 1690 1695

Phe Ile Ala Ala Val Glu Arg Leu Trp Asp Tyr Gly Met Ser Ser
1700 1705 1710

Ser Pro His Val Leu Arg Asn Arg Ala Gln Ser Gly Ser Val Pro
1715 1720 1725

Gln Phe Lys Lys Val Val Phe Gln Glu Phe Thr Asp Gly Ser Phe
1730 1735 1740

Thr Gln Pro Leu Tyr Arg Gly Glu Leu Asn Glu His Leu Gly Leu
1745 1750 1755

Sequence Listing korrekt.txt
Leu Gly Pro Tyr Ile Arg Ala Glu Val Glu Asp Asn Ile Met Val
1760 1765 1770

Thr Phe Arg Asn Gln Ala Ser Arg Pro Tyr Ser Phe Tyr Ser Ser
1775 1780 1785

Leu Ile Ser Tyr Glu Glu Asp Gln Arg Gln Gly Ala Glu Pro Arg
1790 1795 1800

Lys Asn Phe Val Lys Pro Asn Glu Thr Lys Thr Tyr Phe Trp Lys
1805 1810 1815

Val Gln His His Met Ala Pro Thr Lys Asp Glu Phe Asp Cys Lys
1820 1825 1830

Ala Trp Ala Tyr Phe Ser Asp Val Asp Leu Glu Lys Asp Val His
1835 1840 1845

Ser Gly Leu Ile Gly Pro Leu Leu Val Cys His Thr Asn Thr Leu
1850 1855 1860

Asn Pro Ala His Gly Arg Gln Val Thr Val Gln Glu Phe Ala Leu
1865 1870 1875

Phe Phe Thr Ile Phe Asp Glu Thr Lys Ser Trp Tyr Phe Thr Glu
1880 1885 1890

Asn Met Glu Arg Asn Cys Arg Ala Pro Cys Asn Ile Gln Met Glu
1895 1900 1905

Asp Pro Thr Phe Lys Glu Asn Tyr Arg Phe His Ala Ile Asn Gly
1910 1915 1920

Tyr Ile Met Asp Thr Leu Pro Gly Leu Val Met Ala Gln Asp Gln
1925 1930 1935

Arg Ile Arg Trp Tyr Leu Leu Ser Met Gly Ser Asn Glu Asn Ile
1940 1945 1950

His Ser Ile His Phe Ser Gly His Val Phe Thr Val Arg Lys Lys
1955 1960 1965

Glu Glu Tyr Lys Met Ala Leu Tyr Asn Leu Tyr Pro Gly Val Phe
1970 1975 1980

Glu Thr Val Glu Met Leu Pro Ser Lys Ala Gly Ile Trp Arg Val
1985 1990 1995

Glu Cys Leu Ile Gly Glu His Leu His Ala Gly Met Ser Thr Leu
2000 2005 2010

Sequence Listing korrekt.txt
Phe Leu Val Tyr Ser Asn Lys Cys Gln Thr Pro Leu Gly Met Ala
    2015                2020                2025

Ser Gly His Ile Arg Asp Phe Gln Ile Thr Ala Ser Gly Gln Tyr
    2030                2035                2040

Gly Gln Trp Ala Pro Lys Leu Ala Arg Leu His Tyr Ser Gly Ser
    2045                2050                2055

Ile Asn Ala Trp Ser Thr Lys Glu Pro Phe Ser Trp Ile Lys Val
    2060                2065                2070

Asp Leu Leu Ala Pro Met Ile Ile His Gly Ile Lys Thr Gln Gly
    2075                2080                2085

Ala Arg Gln Lys Phe Ser Ser Leu Tyr Ile Ser Gln Phe Ile Ile
    2090                2095                2100

Met Tyr Ser Leu Asp Gly Lys Lys Trp Gln Thr Tyr Arg Gly Asn
    2105                2110                2115

Ser Thr Gly Thr Leu Met Val Phe Phe Gly Asn Val Asp Ser Ser
    2120                2125                2130

Gly Ile Lys His Asn Ile Phe Asn Pro Pro Ile Ile Ala Arg Tyr
    2135                2140                2145

Ile Arg Leu His Pro Thr His Tyr Ser Ile Arg Ser Thr Leu Arg
    2150                2155                2160

Met Glu Leu Met Gly Cys Asp Leu Asn Ser Cys Ser Met Pro Leu
    2165                2170                2175

Gly Met Glu Ser Lys Ala Ile Ser Asp Ala Gln Ile Thr Ala Ser
    2180                2185                2190

Ser Tyr Phe Thr Asn Met Phe Ala Thr Trp Ser Pro Ser Lys Ala
    2195                2200                2205

Arg Leu His Leu Gln Gly Arg Ser Asn Ala Trp Arg Pro Gln Val
    2210                2215                2220

Asn Asn Pro Lys Glu Trp Leu Gln Val Asp Phe Gln Lys Thr Met
    2225                2230                2235

Lys Val Thr Gly Val Thr Thr Gln Gly Val Lys Ser Leu Leu Thr
    2240                2245                2250

Ser Met Tyr Val Lys Glu Phe Leu Ile Ser Ser Ser Gln Asp Gly
    2255                2260                2265

Sequence Listing korrekt.txt
His Gln Trp Thr Leu Phe Phe Gln Asn Gly Lys Val Lys Val Phe
2270 2275 2280

Gln Gly Asn Gln Asp Ser Phe Thr Pro Val Val Asn Ser Leu Asp
2285 2290 2295

Pro Pro Leu Leu Thr Arg Tyr Leu Arg Ile His Pro Gln Ser Trp
2300 2305 2310

Val His Gln Ile Ala Leu Arg Met Glu Val Leu Gly Cys Glu Ala
2315 2320 2325

Gln Asp Leu Tyr
2330

## Claims

1. Modified human factor VIII cDNA wherein mutations are inserted either in the wild-type factor VIII cDNA or in a factor VIII cDNA in which the B-domain is partially or completely deleted and may be replaced by a DNA linker segment, **characterised in that**
A) one or several codons of the human factor VIII cDNA which are not identical with the corresponding codon in the same position of the porcine factor VIII cDNA are substituted by a different codon in such a way that
• when the human sequence contains a codon for a neutral amino acid whereas the porcine sequence contains a codon for a charged amino acid then a codon for an amino acid with the same charge as found in the porcine sequence is introduced into the human sequence;
• when the human sequence contains a codon for a charged amino acid whereas the porcine sequence contains a codon for a neutral amino acid then a codon for a neutral amino acid or a codon for an amino acid of the opposite charge is introduced into the human sequence,
• when the human sequence contains a codon for a charged amino acid whereas the porcine sequence contains a codon for an amino acid with the opposite charge then a codon for an amino acid with the opposite charge is introduced into the human sequence or
B) one or several codons for a charged amino acid which are found in the FVIII cDNA of a hemophilic patient are replaced by a codon for an amino acid of the opposite charge.

2. Recombinant expression vector containing the factor VIII cDNA as claimed in claim 1, **characterised in that** it carries in addition transcriptional regulatory elements for expression in a suitable host cell.

3. Modified biologically active recombinant human factor VIII with improved stability wherein mutations are inserted either in the wild-type factor VIII or in a factor VIII in which the B-domain is partially or completely deleted and may be replaced by a linker, **characterised in that**
A.) one or several amino acids of the human factor VIII which are not identical with the corresponding amino acid in the same position of the porcine factor VIII are substituted by a different amino acid in such a way that
• when the human sequence contains a neutral amino acid whereas the porcine sequence contains a charged amino acid then a charged amino acid with the same charge as found in the porcine sequence is introduced into the human sequence;
• when the human sequence contains a charged amino acid whereas the porcine sequence contains a neutral amino acid then a neutral amino acid or an amino acid of the opposite charge is introduced into the human sequence;
• when the human sequence contains a charged amino acid whereas the porcine sequence contains an amino acid with the opposite charge then an amino acid with the opposite charge is introduced into the human sequence or
B) one or several charged amino acids which are found in the FVIII amino sequence of hemophilic patients are replaced by a codon for an amino acid of the opposite charge.

4. Modified biologically active recombinant human factor VIII as claimed in claim 3, wherein the plasma half life of its activated form is more than 3 minutes, preferably more than 10 minutes and most preferably more than 30 minutes.

5. Modified human factor VIII, **characterised in that** its A2-domain is stabilised by the substitution of one or several amino acids as claimed in claim 3.

6. Process for the recombinant production of a modified human factor VIII as claimed in claim 3 either in cell suspension or on a solid support as a bath cell culture or as a perfusion cell culture with continuous production of a conditioned medium **characterised in that** the factor VIII proteins, which are expressed by a suitable host cell line are purified by chromatographic methods.

7. Process as claimed in claim 6, **characterised in that** the transcription units encoding the modified factor VIII cDNA of claims 1 and 2 contain a dominant selectable marker in order to facilitate the isolation of specific cell clones which have integrated said specific c-DNA into their genome.

8. Host cell line for expression of the factor VIII proteins of claim 3, **characterised in that** it is an animal cell line of vertebrate origin which contains the factor VIII cDNA of claim 1 integrated into its genome.

9. Pharmaceutical composition, **characterised in that it** comprises a modified biologically active recombinant human factor VIII of claim 3.

10. Vector for gene therapy of hemophilia A, **characterized in that** it contains a modified FVIII cDNA as claimed in claim 1.
